# EUROPEAN PATENT APPLICATION

(11) **EP 2 042 141 A1**
(43) Date of publication of application: **01.04.2009**
(21) Application number: 07790449.8
(22) Date of filing: 05.07.2007
(51) Int. Cl.: A61F 13/15, A61F 13/472

(54) **AUXILIARY PAD AND ABSORPTIVE ARTICLE WITH AUXILIARY PAD**

(30) Priority: 05.07.2006 JP 2006186143
(71) Applicant: Uni-Charm Corporation, Ehime 799-0111 (JP)
(72) Inventor: KUDO, Jun, Kanonji-shi Kagawa 769-1602 (JP); HASHINO, Akira, Kanonji-shi Kagawa 769-1602 (JP)
(74) Representative: Sperling, Rüdiger
(86) International application number: PCT/JP2007/063507
(87) International publication number: WO 2008/004639

(57) **Abstract**

An absorbent article having an absorbent body capable following movement of the wear's body. An auxiliary pad (3) is a pad used along with a base absorbent body (2) and has a pad side absorbent body (30) as a pad side absorption layer, a surface side layer (33) as a liquid permeable surface side sheet placed on one side in the thickness direction of the pad side absorption section (30), and pad side engagement sections (37a, 37b) as engagement means placed on the other side of the pad side absorption section (30).

## Description

### TECHNICAL FIELD

The present invention relates to an auxiliary pad and an absorbent article with an auxiliary pad.

### BACKGROUND ART

Conventionally, absorbent articles to absorb a certain liquid such as menstrual blood can be exemplified by an absorbent article that has an absorbent layer to absorb the certain liquid, a liquid permeable surface sheet to cover the skin-side face of the absorbent layer, and a liquid impermeable back sheet to cover the garment-side face of the absorbent layer and is entirely formed into a sheet-shape. Such an substantially sheet-shape absorbent article is used in a state contacted with an excretory part of the certain liquid such as menstrual blood and directly absorbs the certain liquid such as menstrual blood from the excretory part and is variously devised so as not to adhere the menstrual blood, etc. flowing along certain grooves, etc. in wearer's bodies to garments and the like

For example, a wing is provided at both sides of the absorbent article so as to project in the width direction as a means to prevent leakage of menstrual blood, and the menstrual blood is absorbed by way of folding the wing to an underwear side to fix the absorbent article or raising the absorbent body around the excretion area of menstrual blood to the side of the excretory part to make the absorbent body closely contact the excretion area. However, in some cases as well, these absorbent articles cannot appropriately comply with body motions, shape changes of wearer's body surfaces, etc.

On the other hand, an absorbent article has been proposed that has a lower absorbent body fixed to a main body portion of the absorbent article and an upper absorbent article separate from the lower absorbent body, in which an elastic stretchable intermediate sheet, disposed in the longitudinal direction and interposed between the upper absorbent body and the lower absorbent body, pushes up the upper absorbent body to contact with wearer's bodies, thereby improving followablity with respect to an excretory part (e.g., see Patent Document 1).
[Patent Document 1] Japanese Unexamined Patent Application, First Publication No. 2000-152957

### DISCLOSURE OF THE INVENTION

### Problems to be Solved by the Invention

However, the absorbent article in Patent Document 1 has an uppermost face that is flat and wide in the width direction when worn since the upper absorbent body is entirely fixed along the longitudinal direction to the lower absorbent body of sanitary napkin and is fixed to both ends of the lower absorbent body in the width direction. For this reason, due to a certain space occurring between the upper absorbent body and grooves of wearer's bodies, menstrual blood may leak since it is unable to contact the upper absorbent body with excretion areas at groove depths of the wearer's body with no gap.

Moreover, when the wearer's body moves somewhat in a lateral direction, the upper absorbent body can follow the side of the wearer's body by deforming the upper absorbent body itself; however, the upper absorbent body cannot follow motions of the wearer's body or underwear to shift widely in the width direction or motions of the wearer's body or underwear in the longitudinal direction.

In addition, since the upper absorbent body is entirely fixed to the lower absorbent body along the longitudinal direction, when the sanitary napkin is mounted and attached to underwear, the sanitary napkin is attached at a position to which users cannot see and thus it is not necessarily that the upper absorbent body can be always properly attached to contact with body grooves. In a case where the attaching cannot be carried out so as to contact the upper absorbent body with body grooves, leakage may occur since it is possible for a certain space to occur between the upper absorbent body and an excretory part.

The present invention has been made in view of the problems described above, and has an object of the present invention to provide an auxiliary pad having an absorbent body capable of following the movement of the wear's body.

### Means for Solving the Problems

In a first aspect, an absorbent article includes a base absorbent article of substantially oblong-shape; and an auxiliary pad at a skin contacting side of the base absorbent article, in which at least one end portion of the auxiliary pad is engaged to be separable from the base absorbent article, in which the auxiliary pad includes a pad side absorbent layer, a liquid permeable sheet disposed at the surface of the pad side absorbent layer, a pad side engaging portion, disposed at a skin noncontacting side of the auxiliary pad which is opposite to the skin contacting side, that engages the base absorbent article and the auxiliary pad, and a substantially plate-shaped handle portion disposed on at least one end portion in the longitudinal direction of the auxiliary pad.

According to a second aspect, in the absorbent article according to the first aspect, flexural rigidity of the auxiliary pad toward the skin contacting side is 0.05 to 1.7 N.

According to a third aspect, in the absorbent article according to the first aspect, compression hardness (LC) of the auxiliary pad is 0 to 0.8 (-) at the surface of the skin contacting side.

According to a fourth aspect, in the absorbent article according to the first aspect, the auxiliary pad has a first region, extending in the longitudinal direction, at substantially a middle in a width direction, which is perpendicular to the longitudinal direction of the auxiliary pad, and the first region is different in rigidity from a second region, formed at both sides of the first region in the width direction so as to extend in the longitudinal direction.

According to a fifth aspect, in the absorbent article according to any one of the first to the fourth aspects, the pad side engaging portion includes a first engaging portion and a second engaging portion, and the first engaging portion and/or the second engaging portion is configured of a mechanical engaging material.

According to a sixth aspect, in the absorbent article according to the fifth aspect, the first engaging portion and/or the second engaging portion is a hook member, the hook member including a plurality of pins and a base portion to support the plurality of pins, each of the pins being formed so that at least a portion of each pin tilts from the base portion, and the hook member having a higher engaging force when each of the pins is slid toward a direction tilted from the base portion than an engaging force when each of the pins is slid toward the opposite direction.

According to a seventh aspect, in the absorbent article according to the sixth aspect, a tip end of each of the pins is disposed so as to face the inside of the auxiliary pad in the longitudinal direction.

According to an eighth aspect, in the absorbent article according to any one of the fifth to the seventh aspects, the first engaging portion and/or the second engaging portion is restricted in movement toward inside in the longitudinal direction and not restricted in movement toward outside in the longitudinal direction, in a state where the first engaging portion and the second engaging are respectively engaged at one face of the base absorbent article.

According to a ninth aspect, in the absorbent article according to any one of the fifth to the eighth aspects, the first engaging portion and/or the second engaging portion has a shear strength of at least 6 N against an inward force in the longitudinal direction, and a shear strength of no higher than 6 N against an outward force in the longitudinal direction.

### Effects of the Invention

In accordance with the present invention, an auxiliary pad can be provided that includes an absorbent body capable of following the movement of the wear's body.

### BRIEF DESCRIPTION OF THE DRAWINGS

FIG. 1 is a plan view that showing auxiliary pad according to a first embodiment of the present invention;
FIG. 2 is a back side view showing an auxiliary pad according to the first embodiment of the present invention;
FIG. 3A is a cross-sectional view showing an auxiliary pad according to the first embodiment of the present invention shown in FIG. 1;
FIG. 3B is a cross-sectional view showing an auxiliary pad according to the first embodiment of the present invention shown in FIG. 1;
FIG. 3C is a cross-sectional view showing an auxiliary pad according to the first embodiment of the present invention shown in FIG. 1;
FIG. 4 is a cross-sectional view showing an auxiliary pad according to the first embodiment of the present invention in Y-Y direction of FIG. 1;
FIG. 5 is a schematic view showing a position of an absorbent body of an auxiliary pad according to the first embodiment of the present invention;
FIG. 6 is a plan view showing a base absorbent body according to the first embodiment of the present invention;
FIG. 7 is a back side view showing a base absorbent body according to the first embodiment of the present invention;
FIG. 8A is a cross-sectional view showing a base absorbent body according to the first embodiment of the present invention shown in FIG. 6;
FIG. 8B is a cross-sectional view showing a base absorbent body according to the first embodiment of the present invention shown in FIG. 6;
FIG. 8C is a cross-sectional view showing a base absorbent body according to the first embodiment of the present invention shown in FIG. 1;
FIG. 9 is a cross-sectional view in Y-Y direction of FIG. 6 showing a base absorbent body according to the first embodiment of the present invention;
FIG. 10 is a schematic view showing a position of an absorbent body disposed to a base absorbent body according to the first embodiment of the present invention;
FIG. 11 is a perspective view showing an auxiliary pad and a base absorbent body in a worn state according to the first embodiment of the present invention;
FIG. 12 is a plan view showing a packed form of the auxiliary pad according to the first embodiment of the present invention;
FIG. 13 is a perspective view showing an auxiliary pad and a base absorbent body in a worn state according to the first embodiment of the present invention;
FIG. 14 is a perspective view showing an auxiliary pad and a base absorbent body in a worn state according to the first embodiment of the present invention;
FIG. 15 is a perspective view showing an auxiliary pad and a base absorbent body in a worn state according to the first embodiment of the present invention;
FIG. 16A is a longitudinal sectional view and a cross-sectional view that show an auxiliary pad according to a second embodiment of the present invention;
FIG. 16B is a longitudinal sectional view and a cross-sectional view that show an auxiliary pad according to the second embodiment of the present invention;
FIG. 16C is a longitudinal sectional view and a cross-sectional view that show an auxiliary pad according to the second embodiment of the present invention;
FIG. 16D is a longitudinal sectional view and a cross-sectional view that show an auxiliary pad according to the second embodiment of the present invention;
FIG. 17A is a longitudinal sectional view and a cross-sectional view that show an auxiliary pad according to a third embodiment of the present invention;
FIG. 17B is a longitudinal sectional view and a cross-sectional view of an auxiliary pad according to the third embodiment of the present invention;
FIG. 17C is a longitudinal sectional view and a cross-sectional view showing an auxiliary pad according to the third embodiment of the present invention;
FIG. 17D is a longitudinal sectional view and a cross-sectional view of an auxiliary pad according to the third embodiment of the present invention;
FIG. 18 is an enlarged sectional view showing a pad side engagement portion of an auxiliary pad according to a fourth embodiment of the present invention;
FIG. 19 is an enlarged sectional view showing a pad side engagement portion of an auxiliary pad according to a fifth embodiment of the present invention;
FIG. 20A is a back side view of an auxiliary pad and a plan view of a base absorbent body according to a sixth embodiment of the present invention;
FIG. 20B is a back side view of an auxiliary pad and a plan view of a base absorbent body according to the sixth embodiment of the present invention;
FIG. 21 is a plan view showing a packed form of the auxiliary pad according to the sixth embodiment of the present invention;
FIG. 22A is a back side view of an auxiliary pad and a plan view of a base absorbent body according to a seventh embodiment of the present invention;
FIG. 22B is a back side view of an auxiliary pad and a plan view of a base absorbent body according to the seventh embodiment of the present invention;
FIG. 23 is an enlarged sectional view showing a hook member used at a pad side engagement portion of an absorbent article according to the first embodiment of the present invention;
FIG. 24 is an enlarged sectional view showing a hook member used at a pad side engagement portion of an absorbent article according to the first embodiment of the present invention;
FIG. 25 is a plan view showing an auxiliary pad according to the first embodiment of the present invention;
FIG. 26 is a perspective view showing an auxiliary pad according to the first embodiment of the present invention;
FIG. 27 is a perspective sectional view showing an auxiliary pad of an absorbent article according to the first embodiment of the present invention;
FIG. 28A is a sectional view showing an auxiliary pad of an absorbent article according to the first embodiment of the present invention;
FIG. 28B is a sectional view showing an auxiliary pad of an absorbent article according to the first embodiment of the present invention;
FIG. 28C is a sectional view showing an auxiliary pad of an absorbent article according to the first embodiment of the present invention;
FIG. 28D is a sectional view showing an auxiliary pad of an absorbent article according to the first embodiment of the present invention; and
FIG. 28E is a sectional view showing an auxiliary pad of an absorbent article according to the first embodiment of the present invention.

### PREFERRED MODE FOR CARRYING OUT THE INVENTION

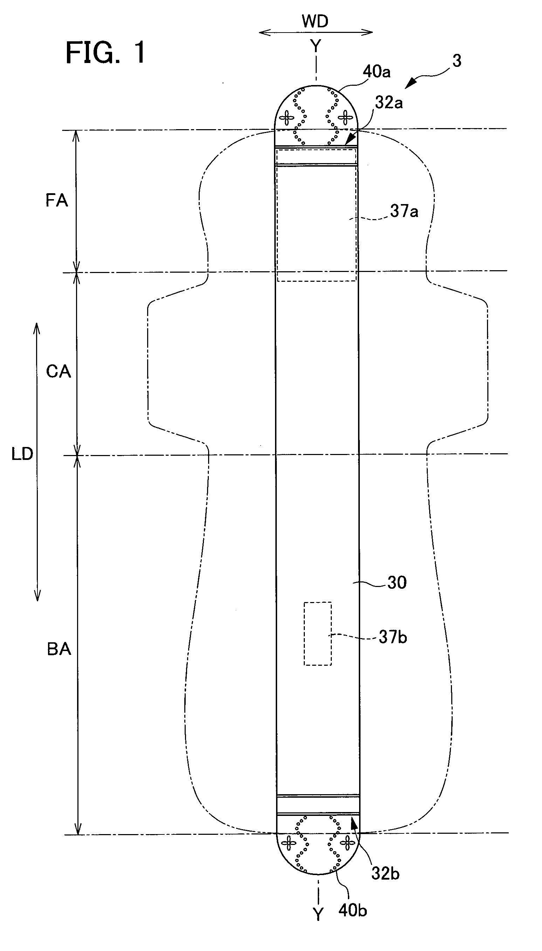

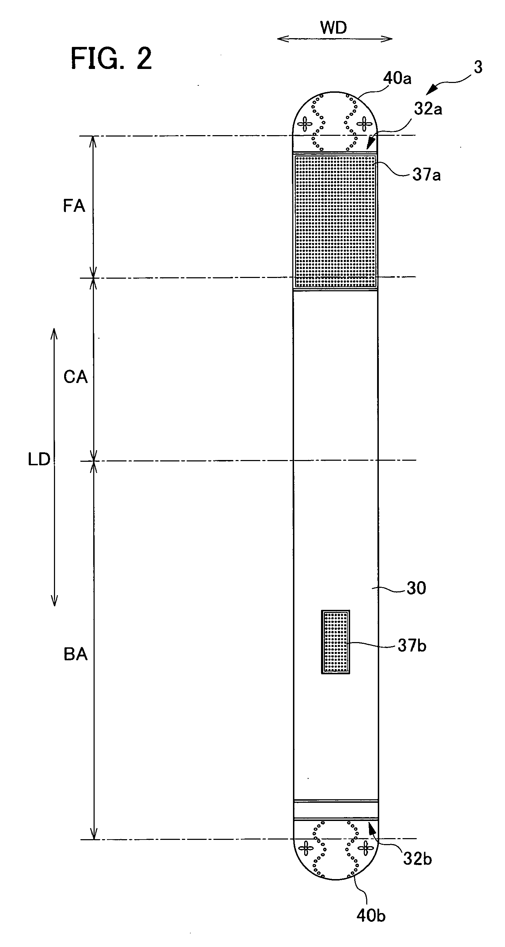

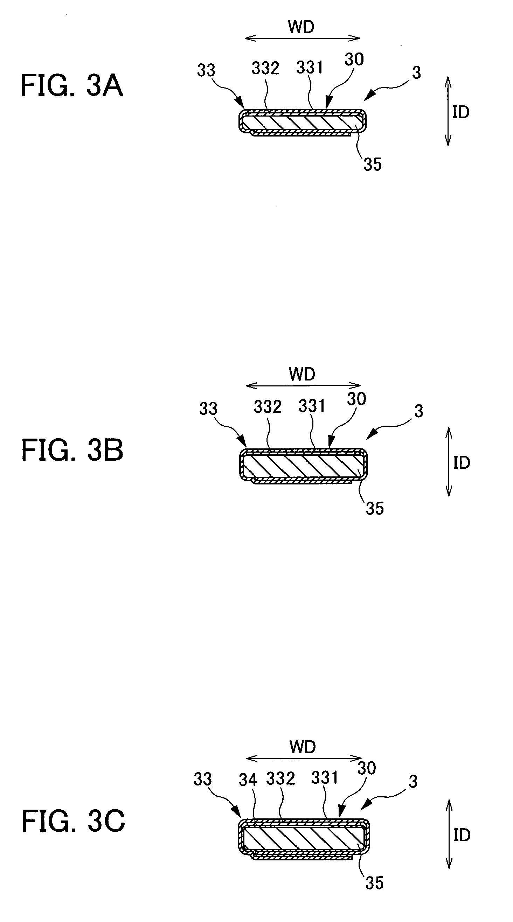

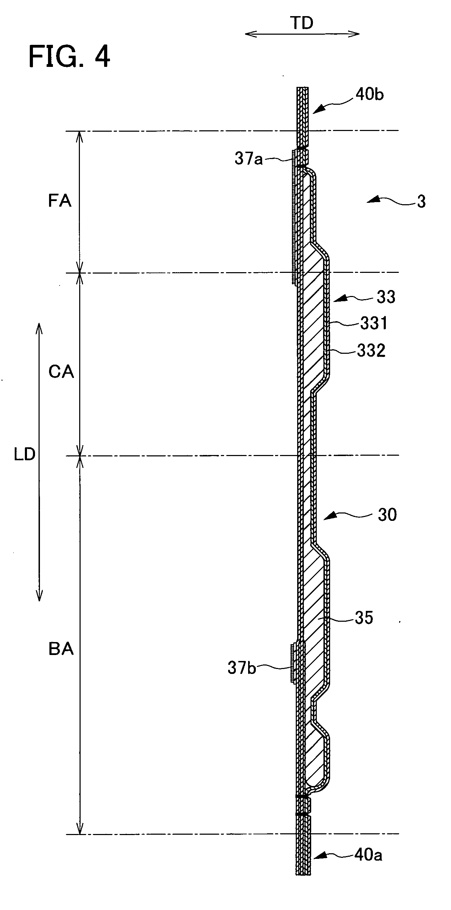

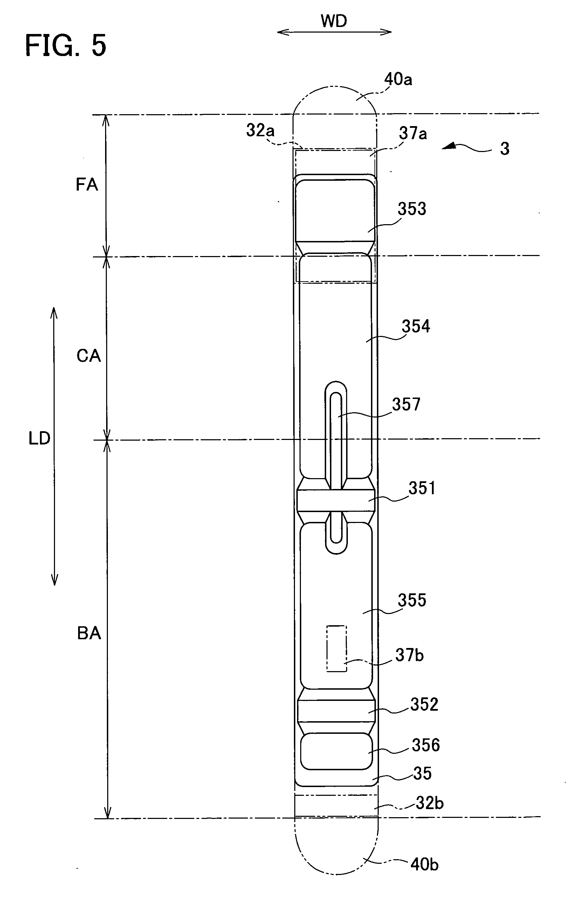

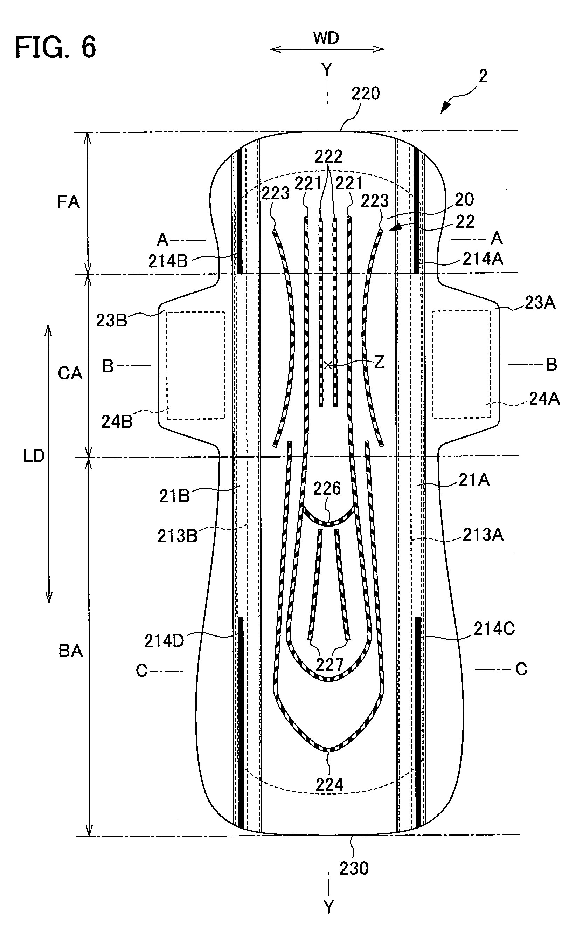

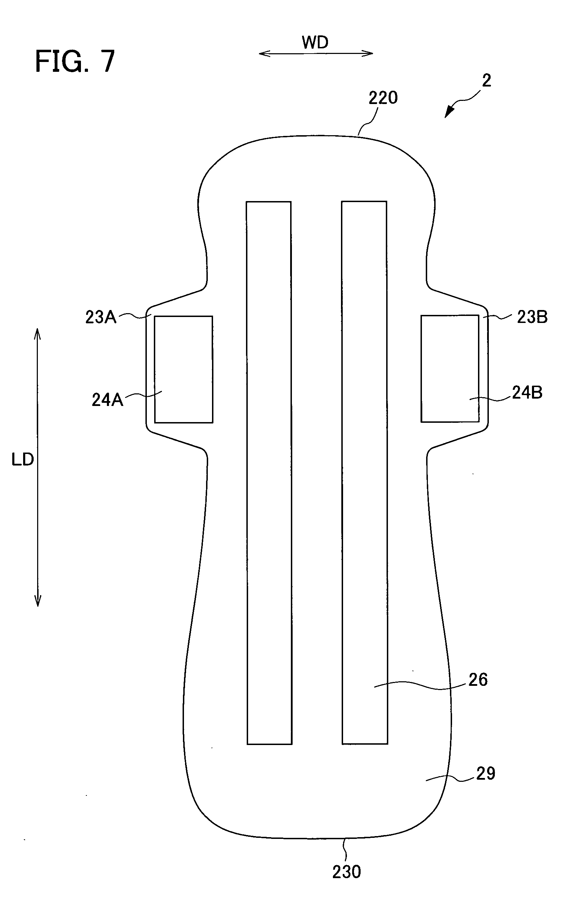

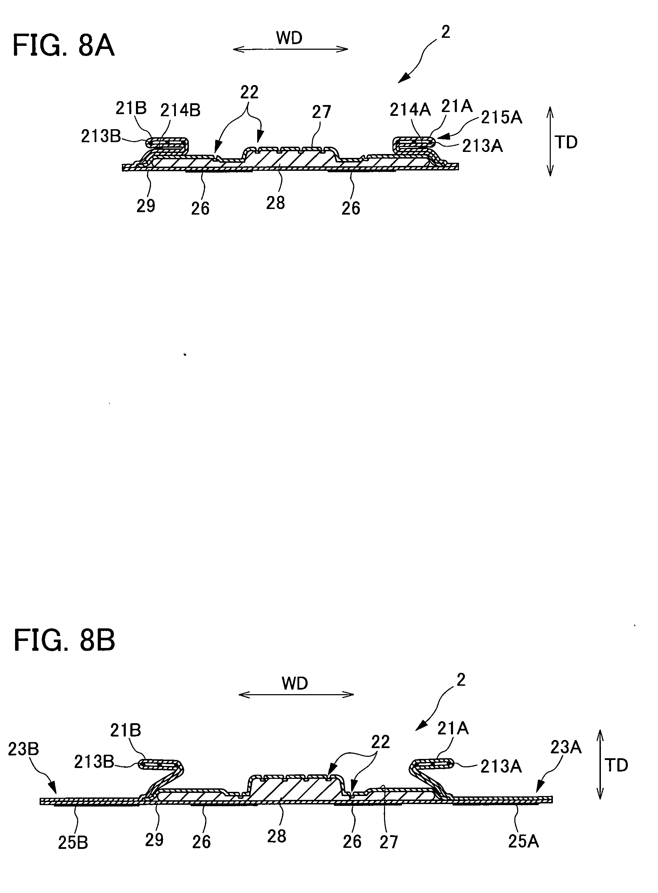

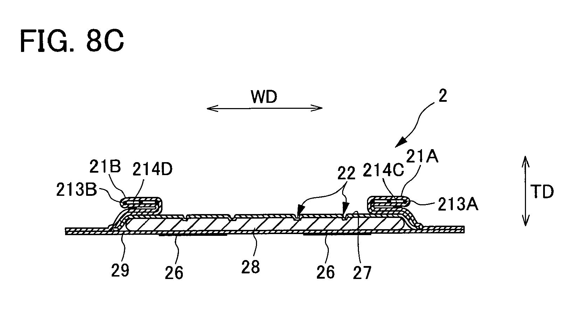

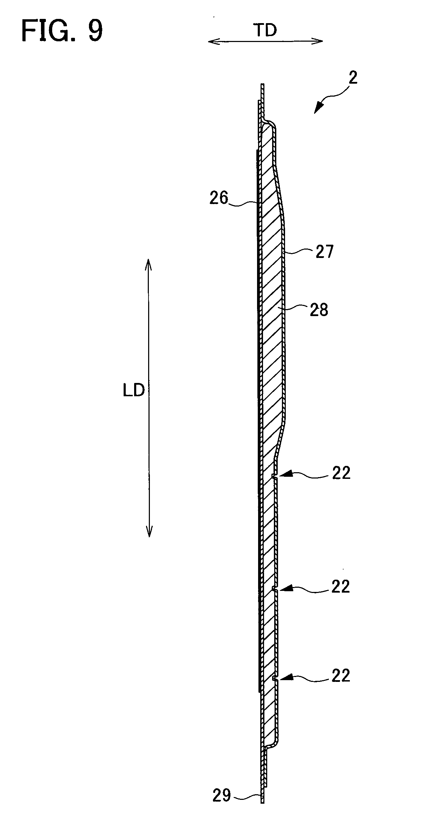

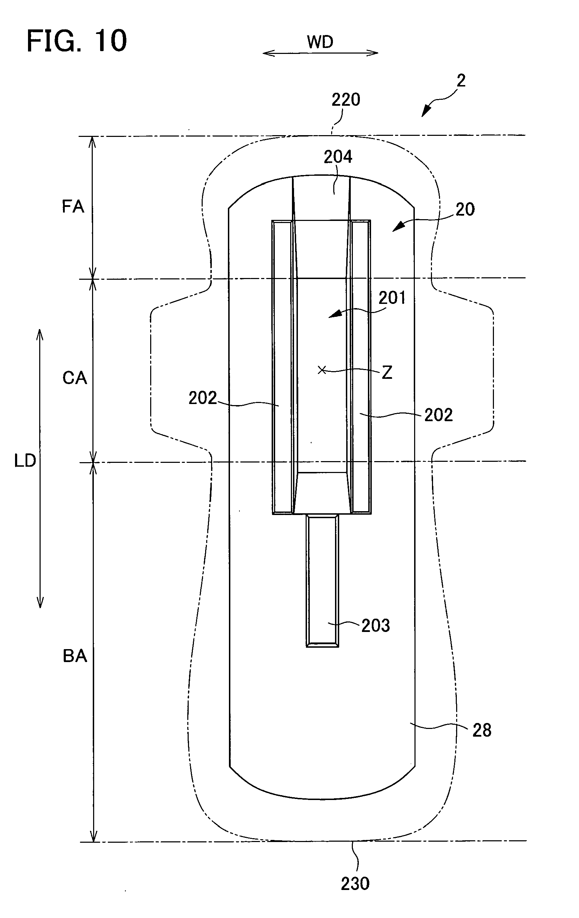

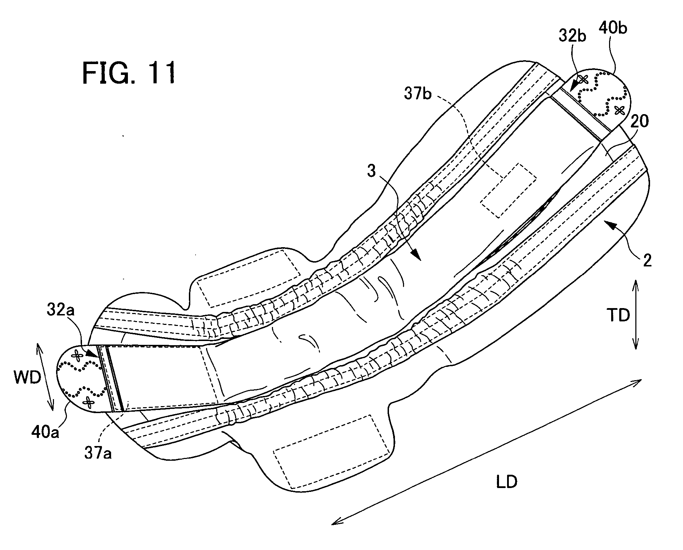

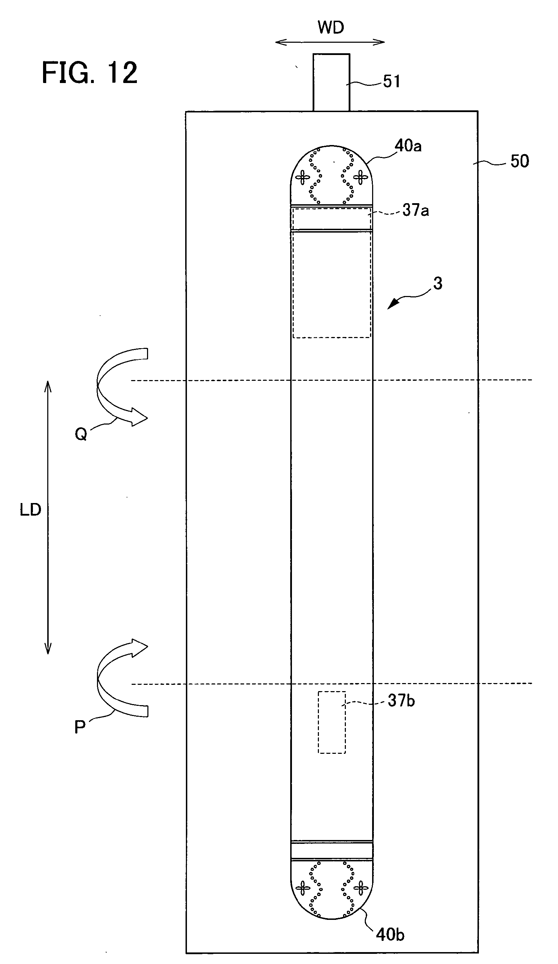

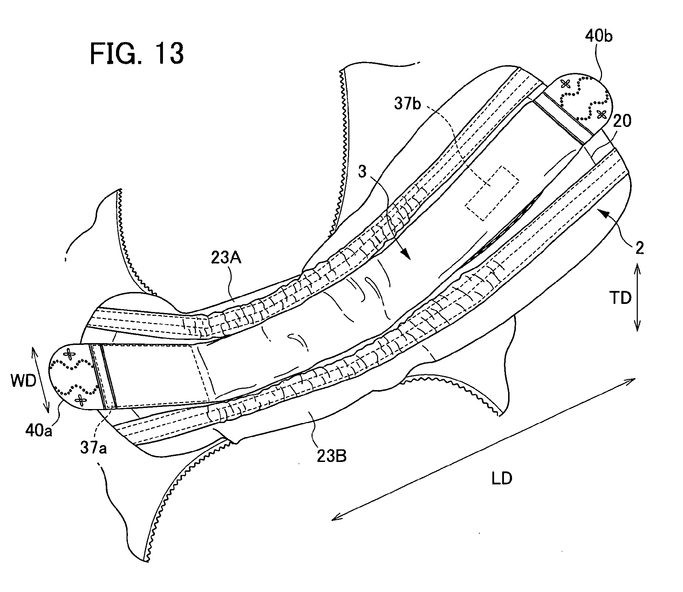

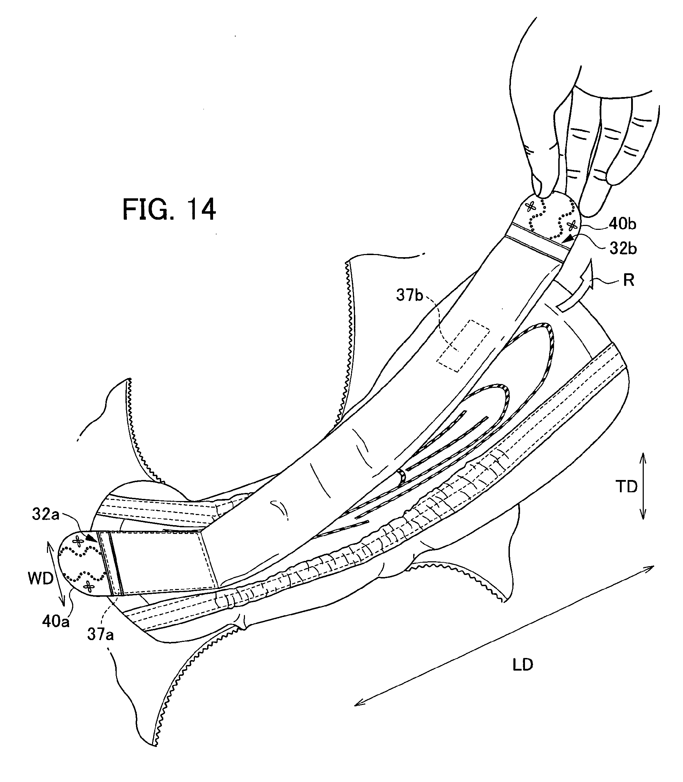

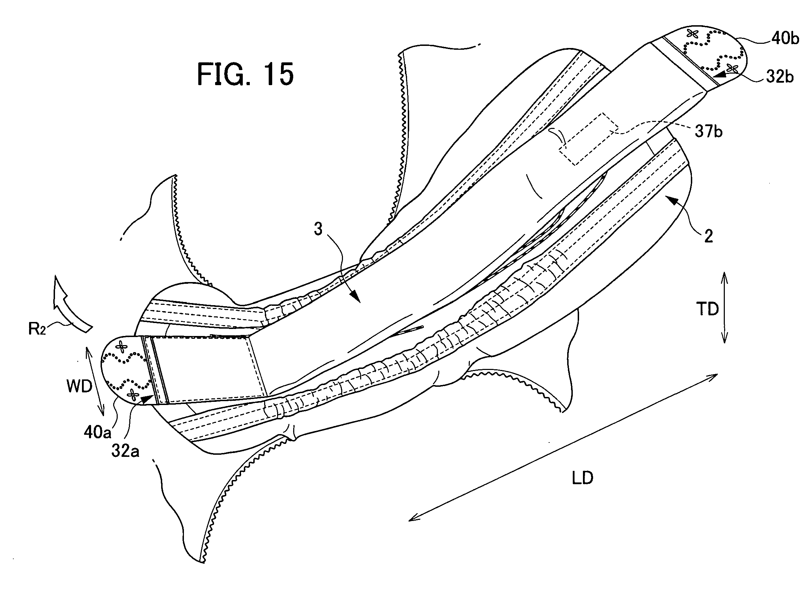

FIG. 1 is a plan view showing an auxiliary pad according to a first embodiment of the present invention. FIG. 2 is a back side view showing an auxiliary pad according to the first embodiment of the present invention. FIG. 3A is a sectional view in A-A direction of FIG. 1 showing an auxiliary pad according to the first embodiment of the present invention. FIG. 3B is a sectional view in B-B direction of FIG. 1 showing an auxiliary pad according to the first embodiment of the present invention. FIG. 3C is a sectional view in C-C direction of FIG. 1 showing an auxiliary pad according to the first embodiment of the present invention. FIG. 4 is a sectional view in the Y-Y direction of FIG. 1 showing an auxiliary pad according to the first embodiment of the present invention. FIG. 5 is a schematic view showing a position of an absorbent body of an auxiliary pad according to the first embodiment of the present invention. FIG. 6 is a plan view showing a base absorbent body according to the first embodiment of the present invention. FIG. 7 is a back side view showing a base absorbent body according to the first embodiment of the present invention. FIG. 8A is a cross-sectional view in the A-A direction of FIG. 6 showing a base absorbent body according to the first embodiment of the present invention. FIG. 8B is a cross-sectional view in the B-B direction of FIG. 6 showing a base absorbent body according to the first embodiment of the present invention. FIG. 8C is a cross-sectional view in the C-C direction of FIG. 6 showing a base absorbent body according to the first embodiment of the present invention. FIG. 9 is a cross-sectional view in the Y-Y direction of FIG. 6 showing a base absorbent body according to the first embodiment of the present invention. FIG. 10 is a schematic view showing a position of an absorbent body disposed to a base absorbent body according to the first embodiment of the present invention. FIG. 11 is a perspective view showing an auxiliary pad and a base absorbent body under use condition according to the first embodiment of the present invention. FIG. 12 is a plan view showing a packed form of the auxiliary pad according to the first embodiment of the present invention. FIG. 13 is a perspective view showing an auxiliary pad and a base absorbent body in a worn state according to the first embodiment of the present invention. FIG. 14 is a perspective view showing an auxiliary pad and a base absorbent body in a worn state according to the first embodiment of the present invention. FIG. 15 is a perspective view showing an auxiliary pad and a base absorbent body in a worn state according to the first embodiment of the present invention.

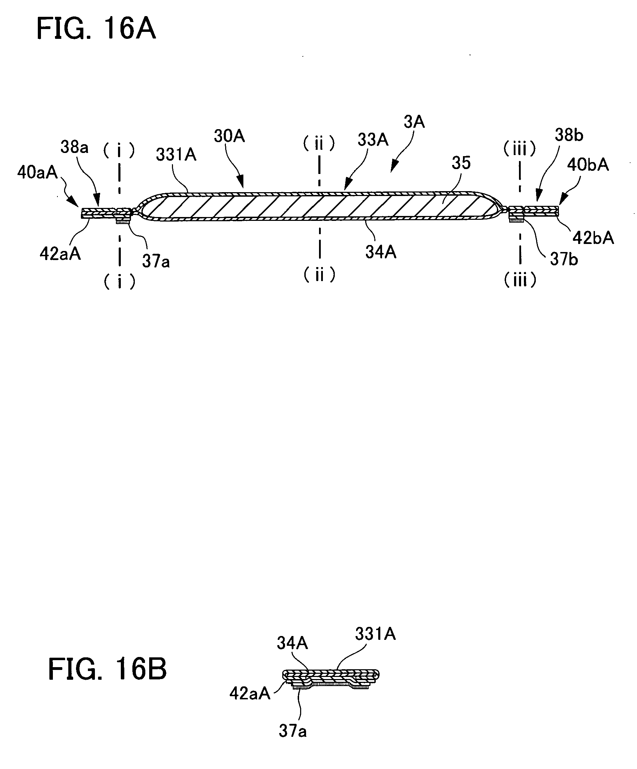

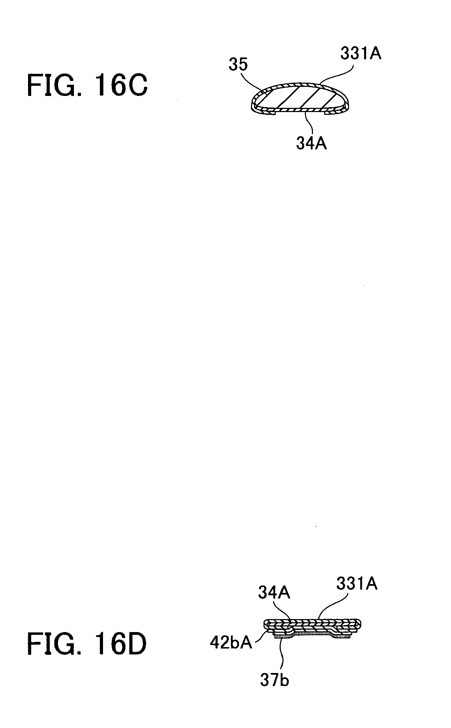

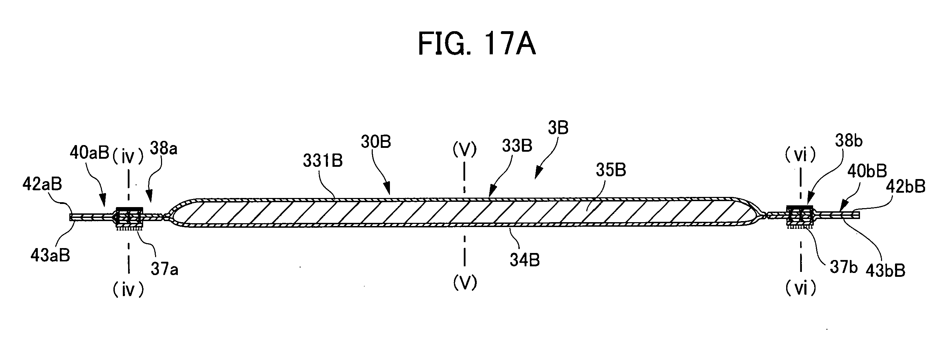

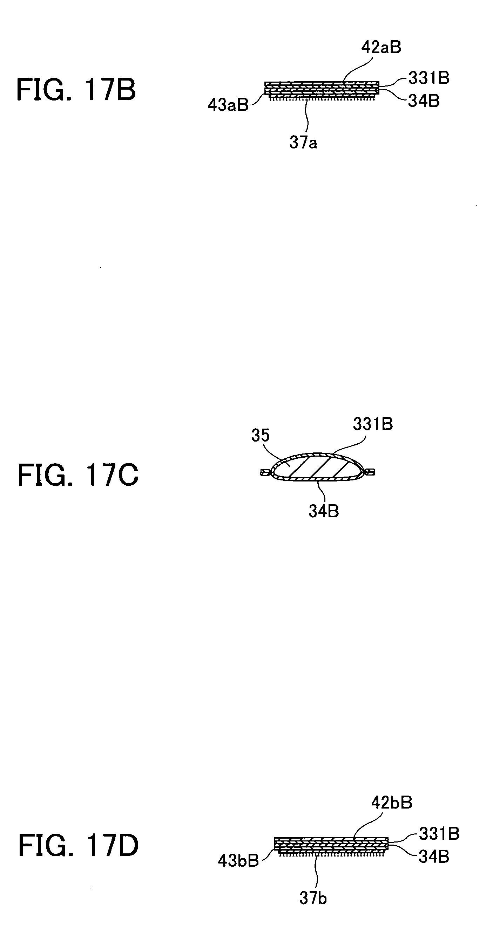

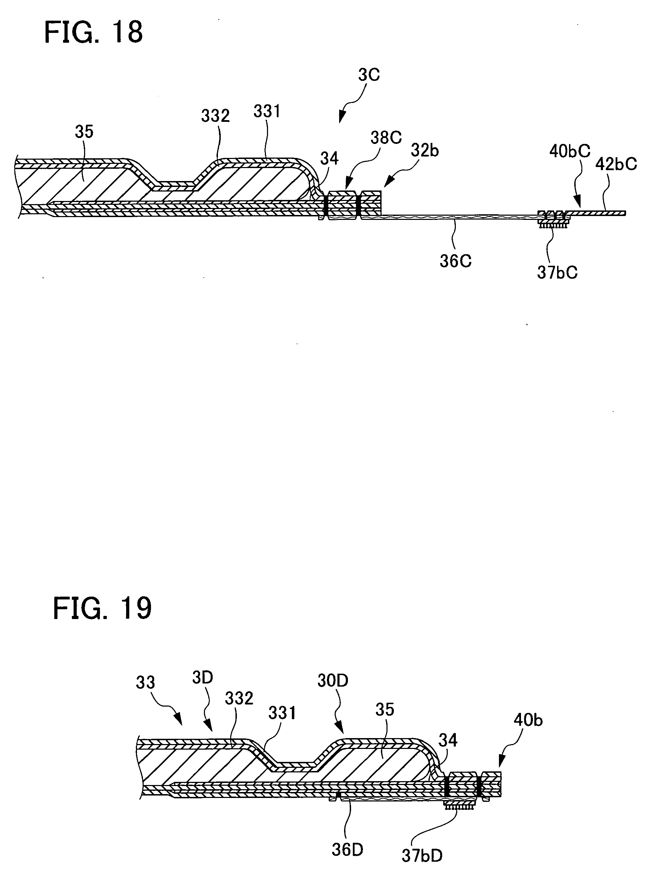

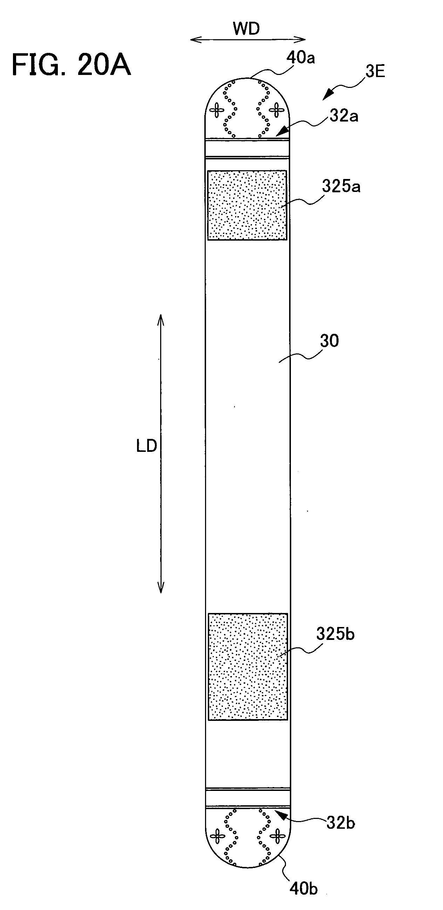

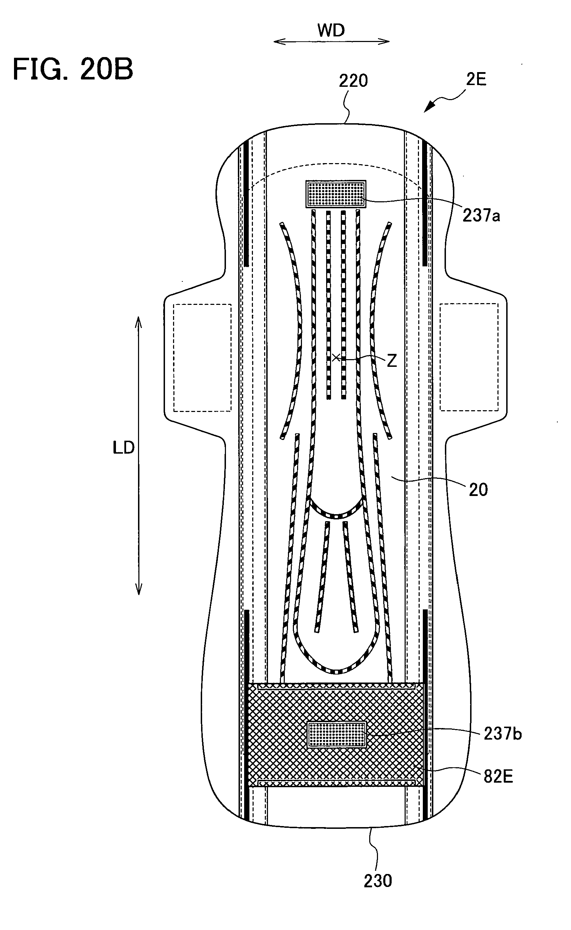

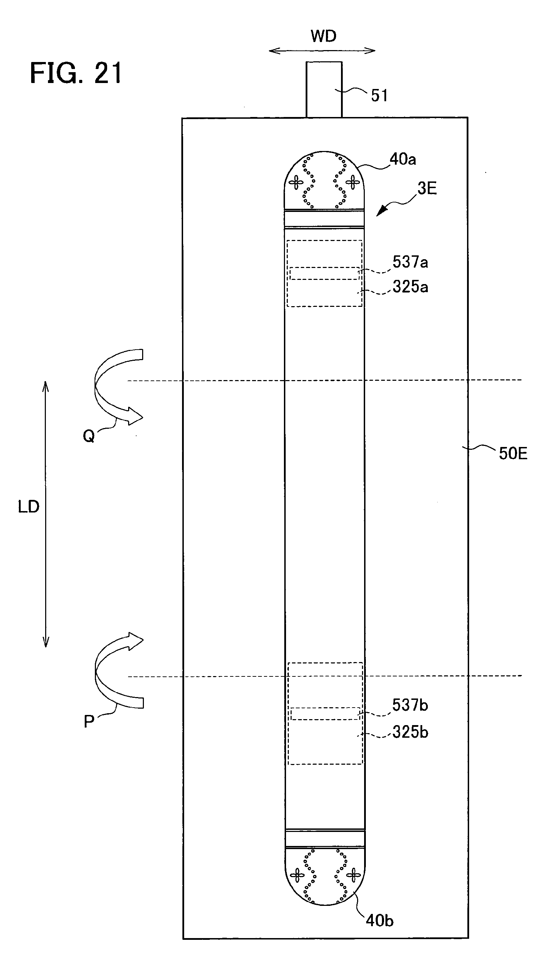

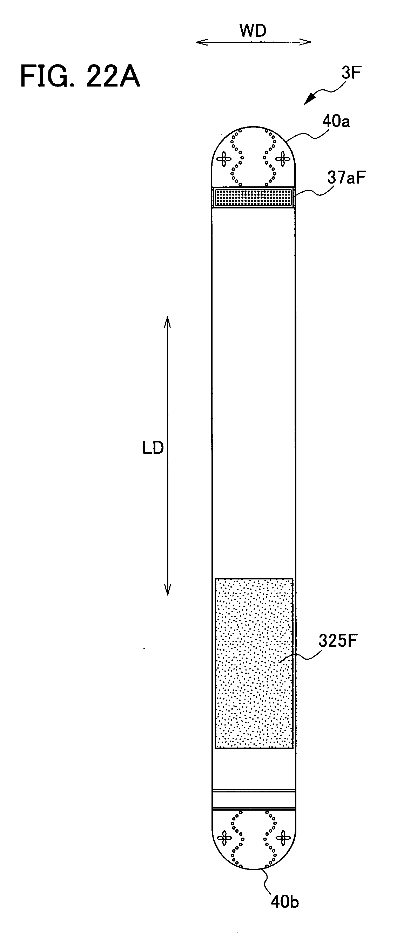

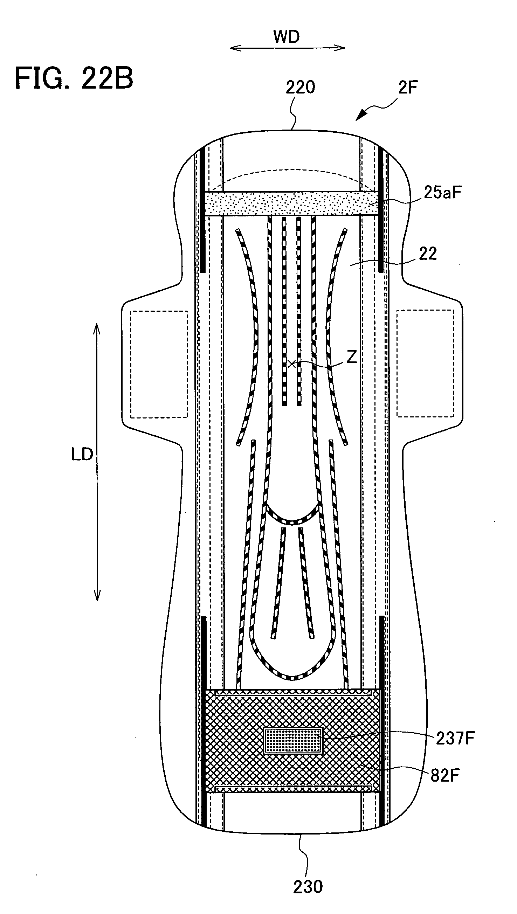

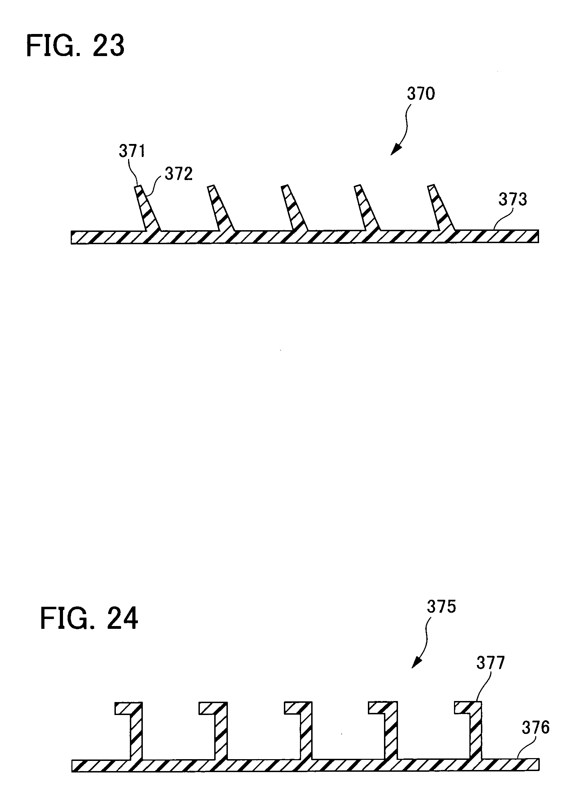

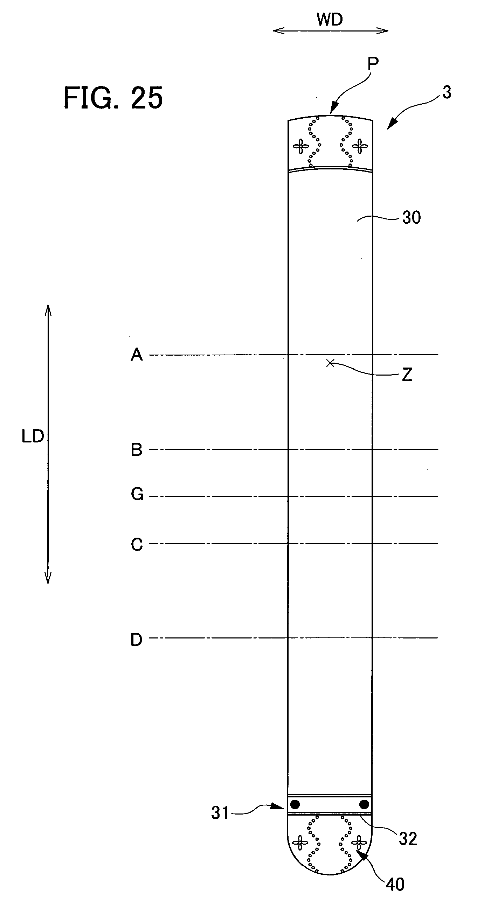

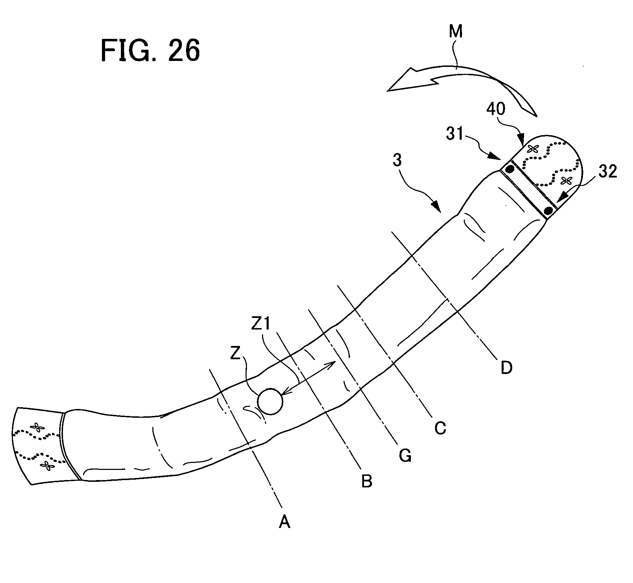

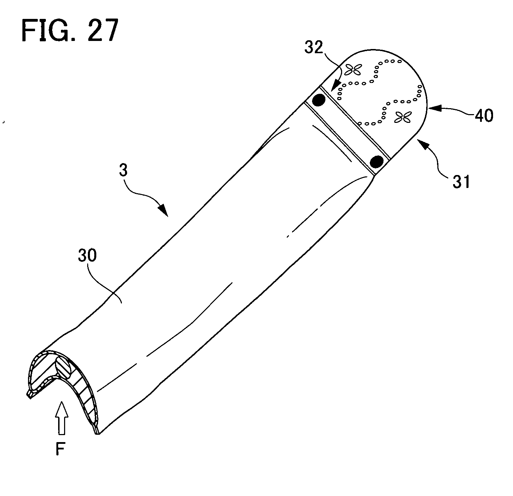

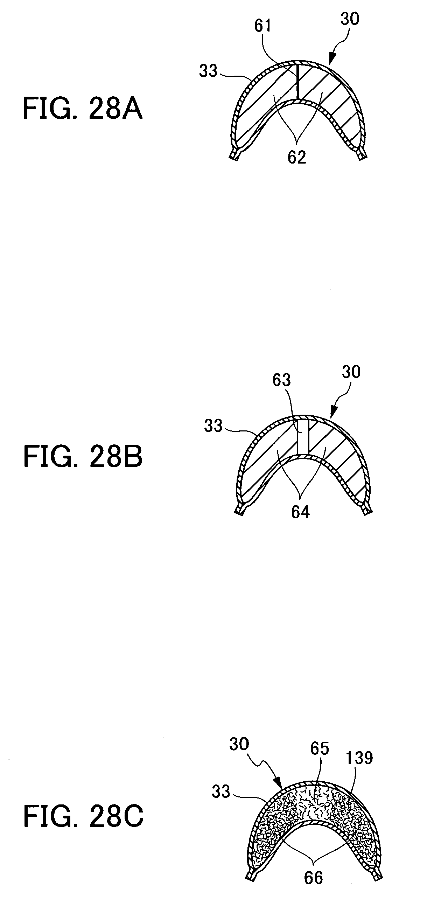

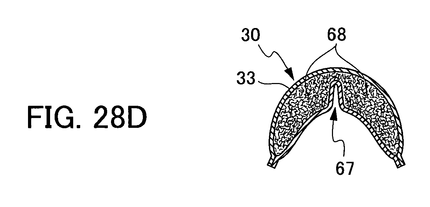

FIG. 16A is a sectional view showing an auxiliary pad 3 in the longitudinal direction LD according to a second embodiment of the present invention; FIG. 16B is a cross-sectional view at (i) of FIG. 16A; FIG. 16C is a cross-sectional view at (ii) of FIG. 16A; and FIG. 16D is a cross-sectional view at (iii) of FIG. 16A. FIG. 17A is a sectional view showing an auxiliary pad 3B in the longitudinal direction LD according to a third embodiment of the present invention; FIG. 17B is a cross-sectional view at (iv) of FIG. 17A; FIG. 17C is a cross-sectional view at (v) of FIG. 17A; and FIG. 17D is a cross-sectional view at (vi) of FIG. 17A. FIG. 18 is an enlarged sectional view showing a pad side engagement portion of an auxiliary pad according to a fourth embodiment of the present invention. FIG. 19 is an enlarged sectional view showing a pad side engagement portion of an auxiliary pad according to a fifth embodiment of the present invention. FIG. 20A is a back side view of an auxiliary pad according to a sixth embodiment of the present invention. FIG. 20B is a plan view of a base absorbent body according to the sixth embodiment of the present invention. FIG. 21 is a plan view showing an auxiliary pad under package presentation according to the sixth embodiment of the present invention. FIG. 22A is a back side view of an auxiliary pad according to a seventh embodiment of the present invention. FIG. 22B is a plan view of a base absorbent body according to the seventh embodiment of the present invention. FIG. 23 is an enlarged sectional view showing a hook member used at a pad side engagement portion of an absorbent article according to the first embodiment of the present invention. FIG. 24 is an enlarged sectional view showing a hook member used at a pad side engagement portion of an absorbent article according to the first embodiment of the present invention. FIG. 25 is a plan view showing an auxiliary pad according to the first embodiment of the present invention. FIG. 26 is a perspective view showing an auxiliary pad according to the first embodiment of the present invention. FIG. 27 is a perspective sectional view showing an auxiliary pad of an absorbent article according to the first embodiment of the present invention. FIG. 28A is a sectional view showing an auxiliary pad of an absorbent article according to the first embodiment of the present invention. FIG. 28B is a sectional view showing an auxiliary pad of an absorbent article according to the first embodiment of the present invention. FIG. 28C is a sectional view showing an auxiliary pad of an absorbent article according to the first embodiment of the present invention. FIG. 28D is a sectional view showing an auxiliary pad of an absorbent article according to the first embodiment of the present invention. FIG. 28E is a sectional view showing an auxiliary pad of an absorbent article according to the first embodiment of the present invention.

### 1. Overview of Absorbent article with Auxiliary Pad

An auxiliary pad 3 and an absorbent article 1 with an auxiliary pad of the present invention are explained in regards to an entire configuration with reference to the auxiliary pad 3 and the base absorbent body 2 of a base absorbent article in the first embodiment of the present invention shown in FIGS. 1 to 11.

### 1.1. Summary

As shown in FIGS. 1 to 11, the absorbent article 1 with an auxiliary pad in this embodiment is configured with an auxiliary pad 3 and a substantially elongated base absorbent body 2 of a base absorbent article.

As shown in FIG. 1, the auxiliary pad 3 is disposed at a wearer's body side and is disposed to contact the excretory part, etc. of wearer's body in use. More specifically, it is disposed in a shape to draw a certain curve along the gluteal cleft.

As shown in FIG. 6, the base absorbent body 2 is disposed on the garment side to absorb a certain liquid such as menstrual blood that cannot be completely absorbed by the auxiliary pad 3. A strip-shape central portion 20 is formed substantially at the center of the base absorbent body 2 in the width direction WD along the longitudinal direction LD of the base absorbent body 2. Wings 23A and 23B are formed at both sides of the absorbent article 1 in the width direction WD, respectively.

Here, the absorbent article 1 with an auxiliary pad has a position Z as the first position to which body excretory part is intended to contact. The position Z is an intersecting point between a center line Y that extends through the center of the absorbent article 1 in the width direction WD along the longitudinal direction LD and a center line B-B that extends through the middle of the wings 23A and 23B in the longitudinal direction LD along the width direction. In other words, the wings 23A and 23B are formed so as to satisfy the positional relation described above. Furthermore, in the base absorbent body 2, the region to dispose the wings 23A, 23B is defined as a central region CA, the region in front of the region to dispose the wings 23A, 23B is defined as a forward region FA, and the region behind the position to dispose the wings 23A, 23B is defined as a backward region BA. Details are as described later.

As shown in FIG. 1, the auxiliary pad 3 includes pad side engagement portions 37a and 37b for an engagement means on at least one end side of the auxiliary pad 3 in the longitudinal direction LD. The auxiliary pad 3 is engaged to the base absorbent body 2 by the pad side engagement portions 37a and 37b. Furthermore, handle portions 40a, 40b are disposed at outer margin of the auxiliary pad 3 in the longitudinal direction LD.

The auxiliary pad 3 engages one of the handle portions 40a and 40b to the base absorbent body 2, and the handle portion of 40a or 40b, disposed at an outer side of another pad side engagement portion of 37a or 37b, is gripped, thereby allowing the auxiliary pad 3 to be disposed so that one side of the engaged auxiliary pad 3 is used as a starting point and another side is disposed along a gluteal cleft of a groove close to excretory parts of wearer's bodies. Details are as described later.

### 1.2. Auxiliary Pad

As shown in FIGS. 1 to 5 and 11, the auxiliary pad 3, which is placed at a side of one face of the base absorbent body 2, is a substantially elongated absorbent body disposed at a body contacting side. The auxiliary pad 3, which can be disposed along the central portion 20 of the base absorbent body 2, includes at least a pad side absorbent portion 30 of a substantially elongated pad side absorbent layer, handle portions 40a and 40b disposed at both sides of the pad side absorbent portion 30 in the longitudinal direction LD, and the pad side engagement portions 37a and 37b to configure an engagement means for engaging the base absorbent body 2 and the auxiliary pad 3.

The auxiliary pad 3 can be completely spaced apart from the base absorbent body 2 and independently operated. Furthermore, the position of the auxiliary pad 3 in a worn state can be appropriately adjusted by way that one of the pad side engagement portions 37a and 37b is engaged to the base absorbent body 2, and then the position of another side is regulated using the other side as a free end. In addition, the position of the auxiliary pad 3 in a worn state can be appropriately adjusted even under in a state where both ends of the handle portions 40a and 40b are gripped.

As shown in FIGS. 3A, 3B, 3C, and 4, the pad side absorbent portion 30 includes a surface layer 33 to be disposed at a skin contacting side, a pad side absorbent body 35, and a back sheet 34 used for a leakage-proof layer to be disposed at the side of the pad side absorbent portion 30 to contact the base absorbent body 2. The pad side absorbent portion 30 is a main member to absorb a certain liquid excreted from excretory parts.

The surface layer 33 is integrally formed by way that a top sheet 331 and a second sheet 332 are disposed to be layered, and then perforated thereto. The second sheet 332 is disposed so as to cover the surface of the skin contacting side of the pad side absorbent body 35.

The top sheet 331 is disposed at the skin contacting side of the second sheet 332 so as to entirely wrap the second sheet 332, the pad side absorbent body 35 described later, and the back sheet 34. The top sheet 331 configures an uppermost face of the pad side absorbent portion 30. Additionally, the top sheets 331 are doubly arranged at the side of the pad side absorbent portion 30 to contact the base absorbent body 2. It should be noted that the top sheet 331 may be of similar material as those of the surface sheet 27 of the base absorbent body 2 described later.

Furthermore, as shown in FIGS. 3A and 4, the edge of a forward region FA side of the pad side of the absorbent portion 30 in the longitudinal direction LD is in a state in which only the top sheet 331 is laminated to be 3 layers. Additionally, a handle portion 40a is formed at an end portion of a side of the forward region FA. It should be noted that, in the portion where the top sheet 331 is laminated to be three layers, it is preferable for each layer to be adhered with a hot melt adhesive.

As shown in FIGS. 3C and 4, the edge of the pad side absorbent portion 30 at the side of the rear region BA in the longitudinal direction LD is in a state laminated to be three layers in a way that the top sheet 331 and the back sheet 34 are disposed to be layered, and then folded in three. Additionally, a handle portion 40b described later is disposed at the edge of the side of the rear region BA. Furthermore, the top sheet 331 and the back sheet 34 are disposed to be layered, and each layer thereof is adhered with a hot melt adhesive at an end portion of the side of the rear region BA.

The second sheet 332 is disposed at the skin contacting side of the pad side absorbent portion 30 so as to cover the surface of the pad side absorbent body 35. Furthermore, the second sheet 332 is disposed to be layered between the top sheet 331 and the pad side absorbent body 35 described later.

It is preferred that the second sheet 332 is formed to be slightly larger than the pad side absorbent body 35. In this embodiment, the length of the second sheet 332 is 300 mm in the longitudinal direction LD and the length thereof is 45 mm in the width direction WD.

Furthermore, the second sheet 332 in this embodiment is formed from an air through non-woven fabric made of a core-sheath fiber having a polypropylene core and a polyethylene sheath of 3.3 dtex and a fiber length of 51 mm, for example. In addition, the basis weight of the second sheet 332 is 20 g/m², for example. It is preferred that the second sheet 332 is formed to have a density higher than that of the top sheet 331. By virtue of the density of the second sheet 332 being higher than that of the top sheet 331, liquid transferability can be enhanced from the top sheet 331. Furthermore, by arrange the top sheet 331 to be stacked in 2 layers, a density gradient can be provided rather than arranging the second sheet 332.

In addition, as shown in FIGS. 3C and 4, the pad side absorbent portion 30 can be provided with a liquid impermeable back sheet 34 used for a leakage-proof layer. The back sheet 34 is at least a portion of the pad side absorbent body 35 at the side to contact the base absorbent body 2 and is disposed at the side of the handle portion 40b.

It is preferred, as shown in FIG. 3C, that the back sheet 34 is disposed to a side surface from the side to contact the base absorbent body of the pad side absorbent body 35, and not at the skin contacting side. Consequently, in a case where a certain liquid excreted at the pad side absorbent portion 30 flows to the side of the handle portion 40b, the liquid can be absorbed into the pad side absorbent body 35. In the back sheet 34, for example, an SMS non-woven fabric made of a liquid impermeable film or hydrophobic fiber can be used that has a basis weight of 24 g/m² and is formed from three layers spunbond, melt blown, and spunbond.

The pad side absorbent core 35 mainly absorbs and holds the evacuated certain liquid. The pad side absorbent core 35 is formed from a ground pulp and a highly absorbent polymer. Here, it is preferred for the ground pulp to be disposed in the pad side absorbent portion 30 to locally vary the basis weight. More specifically, as shown in FIG. 5, for example, the basis weight is 200 g/m² at the region 353 disposed at s side of the end portion 32a, and the basis weight is 500 g/m² at the regions 354, 355, and 356 from the end portion 32a to the portion 32b.

Furthermore, as shown in FIG. 5, nondense portions 351 and 352 are formed at the rear region BA of the pad side absorbent portion 30. The nondense portion 351 is formed at the side of the central region CA of the rear region BA of the pad side absorbent portion 30 along the width direction WD, and the nondense portion 352 is formed at the side of the rear edge 230 of the rear region BA of the pad side absorbent portion 30 along the width direction WD.

The nondense portions 351 and 352 are regions where the basis weight of ground pulp is smaller than other regions. Additionally, the drawn portions 351 and 352 act as a fold starting point when the auxiliary pad 3 is folded. Here, the term "close to the free end" refers to a region from the outer edge of the handle portions 40a and 40b to one-fourth of the total length of the auxiliary pad 3 containing the handle portions 40a and 40b.

Consequently, in a case where the absorbent article 1 is folded such as at the time of individual packaging, wrinkles are prevented for generating that are derived from a curvature difference of an inner side and outer side in a folding step.

Moreover, a region 357 having a basis weight of 200 g/m² is formed longitudinally substantially at the center of the pad side absorbent portion 30 in the width direction WD, at the 80 mm long region 357 from the position slightly shifted to the side of edge 32b of the position Z. The region 357 induces a deformation of the auxiliary pad 3 in a worn state.

The pad side absorbent body 35 is formed so that the length in the longitudinal direction LD is shorter than the length of the top sheet 331 in the longitudinal direction LD. That is, the pad side absorbent body 35 is not disposed at the both sides of the pad side absorbent portion 30 in the longitudinal direction LD, as described above.

The length of the auxiliary pad 3 is, for example, 200 mm to 500 mm in the longitudinal direction LD, and preferably 230 mm to 450 mm. The length of the auxiliary pad 3 in the first embodiment can be exemplified, for example, by 335 mm in the longitudinal direction LD. In this embodiment, the length of the pad side absorbent portion 30 can be exemplified by 280 mm in the longitudinal direction LD, for example.

Furthermore, in this embodiment, more specifically, the length of the pad side absorbent portion 30 in the width direction WD is shorter than the length of the base absorbent body 2 in the width direction WD. It is also preferred for the pad side absorbent portion 30 to have a length capable of contacting along the gluteal cleft in the longitudinal direction LD.

More specifically, the length of the pad side absorbent portion 30 in the width direction WD is 15 mm to 50 mm, for example, and preferably 20 mm to 40 mm. When the width dimension is less than 15 mm, the width is insufficient to maintain a contact with the vaginal opening area, and leaking tends to occur due to generation of gaps between the wearer's body and the napkin. The width dimension of the pad side absorbent portion 30 in the first embodiment can be exemplified by 40 mm, for example.

Furthermore, it is preferred that the pad side absorbent portion 30 has a substantially equal width in the longitudinal direction LD. It is also preferred that the cross-sectional shape of the pad side absorbent portion 30 in the width direction WD has a substantially parallel shape between the skin contacting side and the side to contact the base absorbent body 2.

The auxiliary pad 3 can be formed entirely of a stretchable non-woven fabric.

### 1.3. Handle Portion

As shown in FIGS. 1 and 4, the handle portions 40a and 40b are disposed at both ends of the pad side absorbent portion 30 in the longitudinal direction LD. The handle portions 40a and 40b are a portion to be pinched and pulled by users in order to regulate the position of the auxiliary pad 3 in the attaching step of the auxiliary pad 3 and/or the absorbent article 1 with an auxiliary pad. The handle portions 40a and 40b are formed to project outward in the longitudinal direction LD to a maximum level at the central portion in the width direction WD.

The outer edge portion of the handle portions 40a and 40b is curved. More specifically, the shape is formed into a substantially half circle of which the apex is the intersecting point between an extended line through substantially the center of the auxiliary pad 3 in the width direction WD and the outer edge.

The handle portions 40a and 40b are disposed at the side of the free end 31 and are formed at the region where there exists the side of the free end 31 of the pad side absorbent portion 30, no absorbent body 35 exists in the pad side absorbent portion 30, and only the top sheet 331 and the back sheet 34 extend.

The handle portions 40a and 40b are formed by way of increasing stiffness of the free end portion 32 of the pad side absorbent portion 30. More specifically, the handle portions 40a and 40b are formed by an embossing process to increase rigidity in a region, where the region is the free end 32 of the pad side absorbent portion 30 and only the top sheet 331 and the back sheet 34 extend thereto. The handle portions 40a and 40b are provided with a small-circle emboss portion of gourd-shape, and also another emboss portion of flower-shape is formed at the lateral portion. The emboss portion of gourd-shape functions as an element to guide the position to put fingers when gripping the handle portions 40a and 40b. The handle portions 40a and 40b can be provided with a design by devising the shape of the emboss portion. That is, a guide element or a pattern can be formed on the handle portions 40a and 40b by the embossing process to impart rigidity. In other words, a certain function can be imparted and a design can be provided at one time by the processing to impart rigidity.

The design (guide element) provided to the handle portions 40a and 40b may be anything depending on the shape of the embossed portion. For example, an indication can be provided that prompting to pinch and pull the handle portions 40a and 40b toward a certain direction in the longitudinal direction LD. More specifically, an outward pointing arrow in the longitudinal direction LD to prompt pulling the handle portions 40a and 40b in the longitudinal direction LD, a point to indicate a pinching site, or a combination of certain colors may be used.

The handle portions 40a and 40b are provided with convex and concave portions on surfaces thereof by performing the embossing process. The convex and concave portions can be an indicator for finding the handle portions 40a and 40b that are placed at a position out of sight of the user in a process of attaching the absorbent article 1.

Furthermore, pad side engagement portions 37a and 37b described later are disposed near the handle portions 40a and 40b. The pad side engagement portions 37a and 37b are disposed at a position to follow the movement of the handle portions 40a and 40b. In other words, the handle portions 40a and 40b are disposed at the position where the movement (position) of the pad side engagement portions 37a, 37b can be appropriately regulated.

Furthermore, the handle portions 40a, 40b are disposed so as to project outward from the outer edge 230 of the base absorbent body 2. The portion to project outward from the outer edge 230 of the handle portions 40a and 40b appropriately performs as a handle portion.

It is preferable for the outer margin of the handle portions 40a and 40b to be disposed so as to extend from the rear edge 230 of the base absorbent body 2. More specifically, starting from the rear edge 230 of the base absorbent body 2, a range from 100 mm outward to 50 mm inward, preferably from 60 mm outward to 30 mm inward, and more preferably from 30 mm outward to 20 mm inward, can be exemplified. Users can be assisted in gripping thereof by virtue that the outer margin portion of the handle portions 40a and 40b of the handle portion 40 is disposed to extend from the rear edge 230 of the base absorbent body 2. For example, the handle portions 40a and 40b can be more easily distinguished when the user tries to locate the handle portions 40a and 40b on her back side when attaching the absorbent article 1. Furthermore, the portion to project outward from the rear edge 230 of the handle portions 40a and 40b appropriately functions as a handle portion.

Flexural rigidity (B) of the handle portions 40a and 40b is 0.1 to 1.2 (10⁻⁴ N·m²/m), preferably 0.2 to 1 (10⁻⁴ N·m²/m), more preferably 0.3 to 0.8 (10⁴ N·m²/m). In a case where the flexural rigidity (B) of the handle portions 40a and 40b is less than 0.1 (10⁻⁴ N·m²/m), for example, a predetermined operation may be difficult such as gripping the handle portions 40a and 40b to position the auxiliary pad 3 at a certain portion of the wearer's body. On the contrary, in a case where the flexural rigidity (B) of the handle portion 40 is higher than 10, for example, the handle portions 40a and, 40b may create an uncomfortable feeling during attaching. It is preferable for the handle portions 40a and 40b have a flexural rigidity (B) within the range described above.

Flexure recovery (2HB) of the handle portions 40a and 40b is no higher than 10 (10⁻² N·m/m), preferably no higher than 7, and more preferably no higher than 3 (10⁻² N·m/m). In a case where the flexure recovery (2HB) of the handle portions 40a and 40b is higher than 10 (10⁻² N·m/m), creases may easily be formed when folding back, and may give an unpleasant sensation to a wearer. Therefore, the handle portions 40a and 40b have a flexure recovery (2HB) within the range described above.

Thickness of the handle portions 40a and 40b is 0.5 to 4 mm, preferably 0.7 to 3.5 mm. When the thickness of the handle portions 40a, 40b is less than 0.5 mm, a feeling of anxiety may be imposed on the users as if it cannot withstand the handling of the users to grip and pull the handle portions 40a, 40b. On the contrary, when the thickness of the handle portions 40a and 40b is larger than 5 mm, it may create a foreign-body sensation for the users during use.

### Measuring Method

Measuring methods of the flexural rigidity (B) and flexure recovery (hysteresis: 2HB) of the handle portions 40a and 40b according to the present invention are explained in the following.

An automatic pure bending tester manufactured by Kato Tech Co. (article name, model type "KESFB2-AUTO-A") was used as a measuring instrument. Samples with an adjusted size of 100 mm by 100 mm were used (those with insufficient width were adjusted in 10 mm increments to between 10 mm and 100 mm).

A sample was bent toward the upper side up to a maximum curvature of 2.5 cm⁻¹ by pure bending to always maintain one side under a circular arc at a chuck span of 1 cm, followed by returning to as before, and then the sample was bent toward the back side to a maximum curvature of 2.5 cm⁻¹, followed by returning as before, and the relation between the curvature and bending moment was evaluated.

The flexural rigidity (B) was obtained as a value on a certain hysteresis curve, and expressed as an average gradient between 0.5 and 1.5 cm⁻¹ of the curvature.

The flexure recovery (2HB) is expressed as a hysteresis width of a bending moment M at a curvature of 1.0 cm⁻¹. Greater flexure recovery (2HB) signified worse (lower) flexure recovery.

### 1.4. Engagement Means

As shown in FIGS. 1 and 4, the base absorbent body 2 and the auxiliary pad 3 have pad side engagement portions 37a and 37b to configure the engagement means and the central portion 20 of the base absorbent body 2 as an engaged body. The pad side engagement portions 37a and 37b engage the auxiliary pad 3 at a certain position.

The pad side engagement portions 37a and 37b are disposed near both ends of the pad side absorbent portion 30 of the auxiliary pad 3. The pad side engagement portion 37a is substantially rectangular and disposed from the end portion 32a toward the end portion 32b so as to cover the portion corresponding to the forward region FA at the side of the pad side absorbent portion 30 to contact the base absorbent body 2. More specifically, the pad side engagement portion 37a is disposed at the side of the pad side engagement portion 37a to contact the base absorbent body 2 so as to cover the region from the end portion 32a to the position corresponding to a feet of the wings 23A and 23B of the base absorbent body 2 when the auxiliary pad 3 is overlapped onto the base absorbent body 2.

Furthermore, the pad side engagement portion 37b is substantially rectangular and disposed close to the end portion 32b along the longitudinal direction LD of the auxiliary pad 3. Furthermore, the pad side engagement portion 37b is disposed, for example, at a position separated 1 mm to 120 mm, preferably 5 mm to 100 mm from the end portion 32b of the pad side absorbent portion 30 of the auxiliary pad 3.

In detail, as shown in FIG. 5, it is disposed between the nondense portion 351 of the pad side absorbent body 35 disposed substantially at the center of the pad side absorbent portion 30 in the longitudinal direction LD and the nondense portion 352 disposed at the side of the rear region BA. In more detail, the end portion of the of the end portion 32b side in the pad side engagement portion 37b is disposed so as to be at a position close to an end portion of the end portion 32a side in the nondense portion 352.

The position to dispose the pad side engagement portions 37a and 37b can be situated depending on engaging objects to be an engaged body. For example, when the pad side engagement portion 37b is engaged to underwear as a garment disposed so as to cover the base absorbent body 2 and the auxiliary pad 3, the pad side engagement portion 37b can be disposed so as to traverse the end portion 32b along the width direction WD of the pad side absorbent portion 30.

The material to form the pad side engagement portions 37a and 37b can be appropriately selected depending on the intended engaged body. For example, in a case where engagement to a wearer's body is intended, it is preferred for the material used to be an adhesive material. The adhesive material used can be exemplified by hot melt adhesives and gel adhesives.

Furthermore, in a case where engagement to underwear or the base absorbent body 2 is intended, mechanical engagement materials can be used as the material (member) of the pad side engagement portions 37a and 37b in addition to the adhesives described above. More specifically, hook members can be used as the mechanical engagement materials. In a case where a hook member is used as the material of the pad side engaging portions 37a and 37b, the engagement can be carried out by engaging the hook member to engaged members disposed at a position corresponding to the position to dispose the engaging portion, or to fibers of underwear or a surface material of the base absorbent body 2.

The hook member can be exemplified by hoop and loop fasteners of male type. In this embodiment, it is preferred for a plurality of pins of the hook member to have a certain direction to engage to fibers. The term "to have a certain direction" indicates a state in which the plurality of pins of the hook member 370 is fixed to a substrate of the hook member 370 in a state of inclining to a certain degree, as shown in FIGS. 23 and 24.

More specifically, the hook member 370, as shown in FIG. 23, is a hook member in which a plurality of substantially needle-shaped pins 372 are fixed to incline at a certain angle from a plane surface and aligned. In detail, a tip end portion 371 of the pins 372 configuring the hook member is formed to converge into a substantially needle-shape, and the plurality of pins 372 is formed from the base sheet 373 of the base portion of the hook member 370 so as to incline at a certain angle from vertical direction. In more detail, the plurality of pins 372 represents a condition to be fixed to the base sheet 373 in a state of inclining at a certain angle α rather than vertical to the plane surface. The plurality of pins 372 of the hook member 370 is respectively formed to incline to the left side in FIG. 23.

The hook member 370 with an orientation shown in FIG. 23 undergoes an engagement with an engaged body by way of being pushed to contact the engaged body, thereby engaging each of the pins 372 with the fibers to configure the engaged body. Even when the hook member 370 is applied a force to a direction to incline the plurality of pins 372 in a state of engaging the plurality of pins 372 with fibers, the plurality of pins 372 is unlikely to deform so as to enlarge the inclining angle over 90 degrees. That is, since the engagement of the plurality of pins 372 with fibers is unlikely to be released in this case, the hook member 370 maintains the engagement condition with the engaged body.

Furthermore, the plurality of pins 372 may be of other than the state inclining from the base sheet 373. More specifically, as shown in FIG. 24, such a state is allowable in which the plurality of pins 377 is fixed vertically to the base sheet 376 and only the tip ends of the plurality of pins 377 are bent toward one direction. By way bending all the tip ends of the pins 377 toward a uniform direction, the hook member 375 is formed having directionality so that engagement occurs upon sliding to one direction and the engagement is released upon sliding in the opposite direction.

Consequently, the hook members 370 and 375 engaging with the engaged body are limited for the movement toward the inclining direction of the pins 372 and 377, and are not limited for the movement opposite to the inclining direction of the pins 372 and 377. That is, the hook members 370 and 375 engaging with the engaged body are not moved when a force in a direction to incline the pins 372 and 377 is applied as a certain direction, and the hook members are moved when a force in a direction opposite to the direction to incline the pins 372, 377 is applied.

In a case where a hook member with directionality is used as the pad side engagement portions 37a and 37b, the hook member is disposed so that the direction inclining a plurality of pins faces the inner side of the auxiliary pad 3 in the longitudinal direction LD. Therefore, when the auxiliary pad 3 and the pad side engagement portions 37a and 37b are made to contact the base absorbent body 2, the pad side engagement portions 37a and 37b are pulled toward the direction to incline the pins by a tension generating from flexing of the base absorbent body 2, and thus engagement occurs and movement is limited. Furthermore, when the auxiliary pad 3 is pulled outward, the pad side engagement portions 37a and 37b are pulled to a direction opposite to the direction of incline of the pins, and thus the engagement is released and the movement becomes possible.

In a case where a hook member 370 or a hook member 375 with directionality is used as the pad side engagement portions 37a and 37b, it is preferred that a shear strength of the hook member 370 or hook member 375 with directionality is at least 6 N in the direction of incline of the pins (direction facing the fixed portion 4). On the other hand, it is preferred that the shear strength in the direction opposite to the direction to incline the pins (direction opposite to the fixed portion 4) is less than 6 N. In a case where the shear strength is less than 6 N in the direction to incline the plurality of pins, engagement may be impossible. Furthermore, in a case where the shear strength is higher than 6 N in the direction opposite to the direction to incline the plurality of pins, release of the engagement may be impossible by the pad side engagement portions 37a and 37b.

It should be noted that it is preferred for the engaging force of the pad side engagement portions 37a and 37b not to decrease, even after attaching and detaching multiple times. An engaged body suited for the material used for the pad side engaging portions 37a and 37b may be used for the region in the base absorbent body 2 where it is intended to contact the pad side engagement portions 37a and 37b. Consequently, a decrease in engaging force caused by attaching and detaching of the pad side engagement portions 37a and 37b for multiple cycles can be prevented. For example, when an adhesive is used for the pad side engagement portions 37a and 37b, a mold-releasing film can be disposed. By contacting the pad side engagement portions 37a and 37b with the mold-releasing film before attachment, a decrease in adhesive force due to fibers falling out onto the pad side engagement portions 37a and 37b can be prevented.

Furthermore, in a case where a hook member is used for the pad side engagement portions 37a and 37b, for example, a hook member of female type can be disposed at the region where the base absorbent body 2 contacts before the pad side engaging portions 37a, 37b are attached to the wearer's body. The engaging force of the hook members are unlikely to be degraded after multiple cycles of attachment and detachment; however, the pins configuring the hook members may fall out when releasing the engagement or fibers may be damaged at the sites where hook members contact. By disposing a hook member of female type at the region where the pad side engaging portions 37a and 37b contact before attaching the absorbent article 1, it is possible to prevent the fall out of the pins of hook members or damage to fibers at the sites where hook members contact.

The engaged body to engage the pad side engagement portions 37a and 37b can be the wearer's body, underwear as a garment, or the base absorbent body 2 described later. In a case where the pad side engagement portions 37a and 37b engage the base absorbent body 2, a certain engaging position may be colored at the central portion 20 of the base absorbent body 2 described later. The region colored at the central portion 20 is a base side engaging portion. Furthermore, the shape of the region, in a case where the region is colored, is exemplified by the shape of an engagement member of the pad side engaging portions 37a and 37b, circle marks, star marks, the shape of the auxiliary pad 3, etc. Furthermore, in a case where a mold-releasing film, a hook member of female type, etc. is disposed as the engaged body, the disposed engaged body may be colored. The colored region is a guide element for suggesting the position to dispose the auxiliary pad 3.

### 1.5. Base absorbent Body

As shown in FIGS. 6 to 9, the base absorbent body 2 is formed into a substantially rectangular shape. The base absorbent body 2 includes a liquid permeable surface sheet 27, a liquid impermeable back sheet 29, and a liquid sustainable base side absorbent body 28 disposed between the surface sheet 27 and the back sheet 29. A strip-shape central portion 20 is formed into the base absorbent body 2 substantially at a center in the width direction WD of the base absorbent body 2 and substantially at a center in the width direction WD of the base absorbent body 2 along the longitudinal direction LD. The auxiliary pad 3 is disposed at the central portion 20. Furthermore, gathers 21A and 21B are formed at both sides of the central portion 20 in the width direction WD. The gathers 21A and 21B contact with a groin portion to suppress leakage of menstrual blood and the like that cannot be absorbed by the auxiliary pad 3 and flows along the surface of the wearer's body. Furthermore, wings 23A and 23B are formed respectively at both sides of the base absorbent body 2 in the width direction WD so as to project in an the outward direction of the width direction WD.

In addition, as shown in FIG. 7, the base absorbent body 2 includes, at the back side of another face, a dislocation preventing portion 26 formed substantially at the center in the width direction WD, and wing side dislocation preventing portions 24A and 24B disposed on the wings 23A and 23B formed at both sides in the width direction WD.

The central portion 20 is formed into a strip-shape substantially at the center of the base absorbent body 2 in the width direction WD along the longitudinal direction LD. The central portion 20 is a thin region that configures the base absorbent layer. The central portion 20 is configured from a surface sheet 27, a back sheet 29, and a base side absorbent core 28. The surface sheet 27 and the base side absorbent core 28 are respectively adhered by a hot melt adhesive and bonded by a plurality of compressed grooves 22 formed along the longitudinal direction LD.

In this embodiment, the surface sheet 27 at the central portion 20 is formed of an air through non-woven fabric having a basis weight (basis weight) of 30 g/m². Furthermore, the non-woven fabric is made of a fiber having a polypropylene core and a polyethylene sheath of 2.2 dtex and a fiber length of 51 mm. It should be noted that it is preferred for a hydrophilic oil solution to be coated on the surface of the fiber.

The base side absorbent core 28 is formed by laminating a ground pulp, into which a highly absorbent polymer is mixed, and enveloping thereof by a tissue (not shown). The tissue is formed to be 15 g/m², for example. It is preferred for the ground pulp to be disposed in the central portion 20, as shown in FIG. 6, so that the basis weight is different depending on the place. For example, the ground pulp can be laminated to be 500 g/m² at the peripheral region 201 containing the position Z. Additionally, the ground pulp is laminated to be 100 g/m² at the regions 202 of both sides of the region 201 in the longitudinal direction LD. Furthermore, the ground pulp is also laminated to be 100 g/m² at the regions 203 formed in the region 201 at the side of the rear edge 230 along the longitudinal direction LD. Furthermore, a region 204, where the ground pulp is 300 g/m², is formed in the region 201 at the side of the front edge 220 in the longitudinal direction LD. As for the other region 205, the ground pulp can be disposed to be layered so as to be 200 g/m². Furthermore, the highly absorbent polymer is mixed so that the average basis weight is 10 g/m² for the entire base side absorbent core 28, and is dispersed and disposed in proportion to the basis weight of the ground pulp at each portion of the base side absorbent core 28. This is because the highly absorbent polymer is uniformly dispersed with the ground polymer.

As shown in FIG. 6, a compressed groove 22 is formed at the central portion 20. The compressed groove 22 is formed in a way such that a highly compressed portion and a low compressed portion are alternatively disposed in the longitudinal direction LD. Furthermore, the compressed groove 22 is formed from the forward region FA to the rear region BA in the central portion 20. The length of the compressed groove 22 can be exemplified by 2 mm in the width direction WD. Furthermore, the space between the adjacent compressed grooves 22 can be exemplified by 4 mm in the width direction WD.

As shown in FIG. 6, six compressed grooves are formed at the front region FA. A compressed groove 223 described later is formed outside in the width direction WD. The compressed groove 223 is described later. Compressed grooves 221 and 221 are formed inside the compressed grooves 223 and 223 in the width direction WD. The compressed grooves 221 and 221 are formed from the front region FA to the rear region BA and connected at the rear region BA, and the compressed grooves 221 and 221 form a U-shape compressed groove that is continuous and projects to the side of the rear edge 230. Compressed grooves 222 and 222 are formed inside the compressed grooves 221 and 221 in the width direction WD. The compressed grooves 222 and 222 are formed linearly from the front region FA to the central region CA.

An end portion of the compressed groove 224 described later is formed in the central region CA in addition to the six compressed grooves 221, 222, and 223. The compressed groove 223 is formed from the front region FA to the central region CA to represent a shallow curve projecting inside in the width direction WD. The compressed grooves 223 and 223 are formed so that the space between each other is narrowest at the position corresponding to the position Z in the longitudinal direction LD. At this position, the six compressed grooves 221, 222, and 223 are formed with a substantially equal space in the width direction WD. The compressed groove 224 is as described later.

The compressed groove 224, compressed groove 226, and compressed grooves 227, 227 are formed in the rear region BA in addition to the compressed groove 221 formed continuously from the front region FA described above.

The compressed groove 224 is formed outside the compressed groove 221. More specifically, it is formed so as to surround the compressed groove 221 formed into a U-shape.

The compressed groove 226 is formed inside the compressed groove 221 so as to extend in the width direction WD. More specifically, the compressed groove 226 is formed in the rear region BA at the side of the central region CA so as to connect the compressed grooves 221, 221 spaced apart in the width direction WD. The compressed groove 223 is formed into a shallow curve of U-shape projecting to the side of the rear edge 230 in the longitudinal direction LD.

The compressed grooves 227 and 227 are formed in the region surrounded by the compressed groove 221 and the compressed groove 226 so as to extend in the longitudinal direction LD.

In the rear region BA, three compressed grooves are formed that are formed so as to traverse in the width direction WD. In other words, three compressed grooves are formed in the longitudinal direction LD at the center in the width direction WD.

These compressed grooves 22 are integrally formed by compressing the base side absorbent body 28 and the surface sheet 27, thereby imparting a certain stiffness to the central portion 20. Additionally, in relation to the rigidity of the auxiliary pad 3, the position of the auxiliary pad 3 can be made free from dislocation when the auxiliary pad 3 and the base absorbent body 2 are fixed to underwear and pulled up. That is, such dislocation can be prevented as in a case where the rigidity of the base absorbent body 2 is too low compared to the rigidity of the auxiliary pad 3, the auxiliary pad 3 cannot follow the base absorbent body 2 when bending, thereby resulting in dislocation of the position of the auxiliary pad 3.

As shown in FIGS. 6, 8A, 8B, and 8C, gathers 21A and 21B may be provided at both sides of the central portion 20 along the longitudinal direction LD. Preferably, the gathers 21A and 21B are formed so that at least a part thereof stands up in the thickness direction.

The gathers 21A and 21B are formed at both sides of the central portion 20 along the longitudinal direction LD so that the positions spaced apart 64 mm from each other with a center of the center of the central portion 20 in the width direction WD. The outer side of the gathers 21A, 21B in the width direction WD is not fixed at least in part thereof and forms a free edge.

The gathers 21A, 21B are formed, for example, by way of doubling a non-woven fabric, which is more hydrophobic than the surface sheet 27 of the central portion 20 and has a certain size, and disposing elastic stretchable members 213A and 213B inside the doubled fabric. The elastic stretchable members 213A and 213B can be disposed in multiple, for example, and three thereof can be disposed with a substantially equal space. The elastic stretchable members 213A and 213B are formed, for example, from a thread rubber of 470 dtex and fixed in a state stretched 1.3 times. Consequently, the gathers 21A and 21B can be made to stand by the stretching force of the elastic stretchable members 213A and 213B.

Both edges of the gathers 21A and 21B in the longitudinal direction LD is folded outwardly in the width direction WD and fixed by hot melt adhesives 214A, 214B, 214C, and 214D (FIG. 6).

Additionally, the end portion of each hot melt adhesive 214A, 214B, 214C, and 214D at the inner side in the longitudinal direction LD becomes a standing point of the gathers 21A and 21B and the region between the standing points is a portion is made to a standing state when used.

Furthermore, the gathers 21A and 21B in this embodiment can be prepared from a spunbond non-woven fabric made of a fiber having a polypropylene core and a polyethylene sheath with a basis weight of 22 g/cm².

The dimension of the gathers 21A and 21B can be properly changed depending on the size of the base absorbent body 2. The stress of the elastic stretchable members 213A and 213B can be determined on the basis of thickness, number, and stretched enlargement ratio of the used thread rubber and properly changed depending on the size of the base absorbent body 2. It is important in this case that skin feel is not deteriorated when the gathers 21A and 21B contact with the skin.

The cross-sectional shape of the gathers 21A and 21B in the width direction WD, in the first embodiment, is such that the free edges of the gathers 21A and 21B are disposed so as to face outwardly of the base absorbent body 2 in the width direction WD, as shown in FIG. 3B.

More specifically, the fixed feet of the gathers 21A and 21B is folded inwardly in the width direction WD and also the gathers 21A and 21B are folded at the central portion of the length (height) outwardly in the width direction WD, thereby disposing the edge so as to face outside in the width direction WD. In the present embodiment, the gathers 21A and 21B are configured by folding one time, but is not limited thereto. For example, the free end may be made to have a cross-sectional shape of approximate sigma-shape facing outwardly in the width direction WD of the base absorbent body by folding the gathers 21A, 21B three times. Furthermore, the gathers 21A and 21B may be disposed in a state in which the free end faces inwardly in the width direction WD of the base absorbent body 2.

Furthermore, a back sheet 29 of a liquid impermeable sheet can be disposed on the surface of the base absorbent body 2 at the side opposite to the side to dispose the auxiliary pad 3. By disposing the liquid impermeable back sheet 29, a certain liquid that cannot be absorbed by the base absorbent body 2 can be controlled so as not to adhere to underwear.

Wings 23A and 23B are formed respectively at both sides of the base absorbent body 2 in the width direction WD so as to project in an outward direction of the width direction WD. The wings 23A and 23B, formed of the back sheet 29 of a liquid impermeable sheet and a non-woven fabric to form the gathers 21A and 21B, are formed so as to project in an outward direction in the width direction WD of the base absorbent body 2. The back sheet 29 and non-woven fabric are adhered to each other by a hot melt adhesive. As shown in FIG. 7, wing-side dislocation preventing portions 24A and 24B are provided on the face of the wings 23A and 23B at the side of underwear. Additionally, the base absorbent body 2 can be fixed to underwear by way of folding the wings 23A and 23B to the side of underwear, and adhering the wing-side dislocation preventing portions 24A and 24B to the underwear.

The distance from the position Z to the forward margin 220 of the forward-side periphery in the longitudinal direction LD can be exemplified by 110 mm. Furthermore, the distance to the backward margin 230 can be similarly exemplified by 220 mm.

Here, the length of the central portion 20 of the base absorbent body 2 in the width direction WD is, for example, 70 mm to 120 mm not including the wings 23A and 23B, and preferably 80 mm to 110 mm. In the first embodiment, 110 mm can be exemplified. Furthermore, the length of the base absorbent body 2 containing the wings 23A and 23B is 120 to 200 mm, and preferably 140 to 180 mm. The length containing the wings 23A and 23B in the width direction WD can be, for example, exemplified by 160 mm in the first embodiment. The length of the base absorbent body 2 in the width direction WD is preferably 1.5 to 7 times of the width of the pad side absorbent portion 30, and more preferably 2 to 5 times.

The length of the base absorbent body 2 in the longitudinal direction LD is, for example, 250 to 500 mm, and preferably 270 to 450 mm. Additionally, the length in the longitudinal direction LD can be, for example, exemplified by 330 mm in the first embodiment. Furthermore, the length of the base absorbent body 2 not including the wings 23A and 23B in the width direction WD is, for example, 70 mm to 120 mm, and preferably 80 mm to 110 mm.

The auxiliary pad 3 and the base absorbent body 2 can be produced by the procedures including the following, for example. Initially, in regards to the base absorbent body 2, the surface sheet 7 and the base side absorbent body 28 are disposed to be layered, and treated for pressure bonding from the side of the base side absorbent body 28 by an embossing roller with a certain pattern, thereby forming the compressed groove 22 and also pressure-bonding to each other. Moreover, a liquid permeable back sheet 29 is disposed to be layered on the face of the base side absorbent body 28 opposite to the surface sheet 27, and bonded by a hot melt adhesive thereby to form the base absorbent body 2.

Furthermore, an embossing process is carried out at a region near the edges 32a and 32b of the auxiliary pad 3, thereby forming the handle portions 40a and 40b. Moreover, the pad side engagement portions 37a and 37b are disposed near the edges 32a and 32b.

Additionally, the pad side engagement portions 37a and 37b of the auxiliary pad 3 are engaged with the central portion 20 of the base absorbent body 2.

### 1.6. Package Presentation of Auxiliary Pad 3

As shown in FIG. 12, the auxiliary pad 3 is packaged in accordance with a substantially rectangular sheet. Additionally, an engaging sheet 51 is disposed at one end portion of one side of the packaging sheet 50 in the longitudinal direction LD and substantially at the center in the width direction WD. The auxiliary pad 3 is disposed on the packaging sheet 50 at another side and substantially at the center in the width direction WD.

The packaging sheet 50 can be exemplified to be formed by an SMS non-woven fabric, for example. When the pad side engaging portions 37a and 37b of the auxiliary pad 3 is formed by a hook member, the pad side engagement portions 37a and 37b can be engaged to the packaging sheet 50. Furthermore, a loop member may be disposed, for example, at a region to contact the pad side engaging portions 37a and 37b of the packaging sheet 50, and engage the pad side engagement portions 37a and 37b of the auxiliary pad 3.

Alternatively, in a case where the pad side engaging portions 37a and 37b are formed by an adhesive, the auxiliary pad 3 is detachably adhered to the region to contact the pad side engagement portions 37a and 37b of the packaging sheet 50 by coating a small amount of non-tacky hot melt adhesive. Furthermore, a mold-releasing film or a mold-releasing material may be disposed at the region to contact the pad side engagement portions 37a and 37b of the packaging sheet 50, thereby engaging the pad side engagement portions 37a and 37b.

After engaging the auxiliary pad 3 to the packaging sheet 50, the packaging sheet 50 along with the auxiliary pad 3 is folded to the side of the auxiliary pad 3 in the order of the first folding P and the second folding Q as shown in FIG. 12. Additionally, the engagement is carried out by the engaging tape 5 under the folded state. Moreover, sealing is carried out for the side periphery in the folded state.

### 1.7. Physical Property of Auxiliary Pad

### 1.7.1. Flexural Rigidity

Due to the auxiliary pad 3 being used to directly contact the wearer's body, flexural rigidity thereof may lead to an uncomfortable feeling. For example, as shown in FIG. 26, although the auxiliary pad 3 deforms in use into a certain curve toward the surface side of the wearer's body side in this case, when there exist a significant rigidity, draping property becomes poor as well as becoming difficult to fill gaps between the auxiliary pad 3 and the wearer's body at grooves close to the body's excretory part and the like. In other words, in a case where the flexural rigidity is at least a certain value, the auxiliary pad 3 cannot follow appropriately the wearer's body shape, and menstrual blood and the like may leak from the gaps formed between the wearer's body. That is, it is preferred for the flexural rigidity of the pad side absorbent portion 30 toward the upper side to be lower than a certain value.

In this embodiment, for example, the flexural rigidity of the auxiliary pad 3 opposite to the side of the base absorbent body is 0.05 to 1.7 N, preferably 0.1 to 1.5 N, and more preferably 0.2 to 1.2 N. When the flexural rigidity is lower than 0.05 N, operability may be inferior during attaching since the auxiliary pad 3 is excessively soft. Furthermore, when the flexural rigidity is higher than 1.7 N, comfortability with the wearer's body may be deteriorated.

Furthermore, it is preferable for the flexural rigidity at the central region, from the position Z shown in FIG. 25 or 26 to the position of the auxiliary pad 3 spaced apart 100 mm toward the side of the free edge 31, to be 0.1 to 1.5 N, and preferably 0.2 to 1.0 N. It is preferable for the central region to have a moderate softness since grooves of the body are particularly deep.

Preferably, the difference between the flexural rigidity at a certain position of the auxiliary pad 3 and the flexural rigidity at another position spaced apart from the certain position in the longitudinal direction LD is no higher than 1.2 N, and preferably 1.0 N. For example, when the auxiliary pad 3 has a site where the flexural rigidity is considerably different between adjacent regions in the longitudinal direction LD, large deformations occur at the site, and the comfortability with the wearer's body may be impaired in the auxiliary pad 3. Furthermore, waste such as menstrual blood may leak from the largely deformable site (where comfortability with the wearer's body is poor). It is, therefore, preferred that the difference between the flexural rigidity at a certain position of the auxiliary pad 3 and the flexural rigidity at the other positions spaced apart from the certain position in the longitudinal direction LD is within the above described range. For example, any bending positions of the bending positions A to D in FIG. 25 or 26 represent the difference of flexural rigidity of no higher than 1.2 N at the adjacent bending positions of A to D.

The value of flexural rigidity of the auxiliary pad 3 is 0.1 to 2 times, preferably 0.2 to 1.5 times, and more preferably 0.3 to 1.2 times the value of flexural rigidity of the base absorbent body 2 to the side of the auxiliary pad 3. For example, when underwear is pulled up in a state where the base absorbent body 2 is attached to inside of the underwear or the like, the auxiliary pad 3 and the base absorbent body 2 are curved in an overlapped state fitting along the underwear shape. Therefore, when the flexural rigidity of the auxiliary pad 3 is at least two times the flexural rigidity of the base absorbent body 2, it may be difficult to curve the auxiliary pad 3 and the base absorbent body 2 in combination. Consequently, the auxiliary pad 3 and the base absorbent body 2 may space apart from each other prior to pulling up the underwear to contact the wearer's body. In this case, the auxiliary pad 3 may not be disposed appropriately to a certain position (e.g., excretory part) due to dislocation upon pulling up. When the value of flexural rigidity of the auxiliary pad 3 is in the range on the basis of the value of flexural rigidity of the base absorbent body 2 to the side of the auxiliary pad 3, the spacing between the auxiliary pad 3 and the base absorbent body 2 can be regulated. It is preferred since the auxiliary pad 3 and the base absorbent body 2 can be curved in combination, for example.

Here, the flexural rigidity of the auxiliary pad 3 and the base absorbent body 2 of the present invention can be measured by the measuring method described below.

### Measurement of Flexural Rigidity

A digital force gauge "FGC-xB series", article name, model FGC-2B, manufactured by Nidec-Shimpo Co. was used for measuring the flexural rigidity.

The measuring procedures are as follows.
(1) A hot melt adhesive, coated on the dislocation preventing portion 26 of the base absorbent body 2 of a measuring object, is made to be non-adherent using Siccarol, and then wings 23A and 23B and gather are cut. The wings 23A and 23B are cut at the feet of the main body and the wings 23A and 23B. The gather is made to be cut at a plurality of sites thereof in the longitudinal direction LD so as to be free of stress by way of cutting the stretchable rubber. In this embodiment, the auxiliary pad 3 is measured without the processing described above.
(2) The measuring object is cut so that the length is 50 mm from a measuring site to the side to contact with a terminal of the digital force gauge.
(3) The measuring object is put on a table (back side facing upward) so as to extend 50 mm therefrom. At this time, the measuring site (place to be bent) of the measuring object is matched with the edge of the table described above. That is, the measuring sample is set to extend 50 mm from the table edge.
(4) At this time, when the measuring object bends downward by its own weight, filter paper is laid on the downside of the measuring object to prevent the bending by its own weight.
(5) A clog of 2 kg is put on to match the edge thereof with the measuring site of the measuring object.
(6) The digital force gauge is driven downward vertically at a rate of 200 mm/min to the position of 5 mm from the table edge.
(7) The maximum load at lowering by 5 mm is recorded. When filter paper is used, the flexural rigidity is determined by subtracting the flexural rigidity of the filter paper from the measured value.

### 1.7.2. Compression Hardness

Furthermore, since the pad side absorbent portion 30 directly contacts excretory areas of the wearer's body, it is desirable to have compression hardness within a certain range at the contacting surface side.

For example, the compression hardness (LC) at the surface of the auxiliary pad 3 opposite to the side of the base absorbent body 2 is 0 to 0.8 (-), preferably 0.4 to 0.75 (-) or less, and further 0.70 (-) or less. When the compression hardness (LC) is 0.4 (-) or less, change in the thickness of the auxiliary pad 3 relative to pressure change becomes large, for example, and absorbability may be impaired due to higher density of the auxiliary pad 3. When compression hardness (LC) is above 0.8 (-), for example, a rigid feeling may be given to the user, thereby decreasing comfortability with the wearer's body. It is, therefore, preferred for the compression hardness (LC) at the surface of the auxiliary pad 3 opposite to the side of the base absorbent body 2 to be within the range. Furthermore, the compression hardness (LC) at the surface of the auxiliary pad 3 opposite to the side of the base absorbent body 2 is the compression hardness (LC) from the surface of the auxiliary pad 3 at the wearer's body side (skin side) toward the side of the base absorbent body 2.

In addition, the compression hardness (LC) at the central region is 0.3 to 0.75 (-), and preferably 0.4 to 0.7 (-). Since the central region is a region that contacts with body grooves and the like so as to fit thereto in particular, due to those having compressive softness being likely to contact thereto at groove depth, it is preferred that the compression hardness (LC) at the central region is within the range.

The value of the compression hardness (LC) at the surface of the auxiliary pad 3 opposite to the side of the base absorbent body 2 is 0.1 to 2 times, preferably 0.2 to 1.5 times, and more preferably 0.3 to 1.2 times on the basis of the value of the compression hardness (LC) of the base absorbent body 2. For example, in a case where the auxiliary pad 3 is used while not spacing apart from the base absorbent body 2 (auxiliary pad 3 does not contact along with fitting body grooves), and in a case where the value of the compression hardness (LC) of the pad side absorbent portion 30 is significantly different from the value of the compression hardness (LC) of the base absorbent body 2, for example, and in a case where the value of the compression hardness (LC) of the auxiliary pad 3 is very large, the auxiliary pad 3 may be embedded in the base absorbent body 2 making it difficult to contact the auxiliary pad 3 properly with the wearer's body.

### 1.7.3. Compression Recovery Rate

Compression recovery rate (RC) of the auxiliary pad 3 at the surface opposite to the side of the base absorbent body 2 is at least 30%, and preferably at least 35%. When the compression recovery rate (RC) is at least 30%, for example, it is preferred for maintaining easily a so-called fit state, since even when the auxiliary pad 3 enters into body grooves and is compressed by pressure, the shape is likely to recover. Here, the compression recovery rate (RC) of the auxiliary pad 3 at the surface opposite to the side of the base absorbent body 2 is the compression recovery rate (RC) when the auxiliary pad 3 is compressed from the surface of the wearer's body side (skin side) toward the side of the base absorbent body 2.

The compression recovery rate (RC) at the central region of a region from the position where the auxiliary pad 3 contacts the excretory part to the position of the auxiliary pad 3 spaced apart 100 mm to the side of the free edge 31 is at least 35%, and preferably at least 40%. Since the central region is a region where contacting with body grooves and the like so as to fit thereto in particular, even when compressed by pressure, a so-called fit state tends to be maintained since the shape is easily recovered. Preferably, the compression recovery rate (RC) at the central region is within the range described above.

### Measurement of Compression Hardness and Compression Recovery

Compression hardness (LC) and compression recovery (RC) in the present invention can be measured by the measuring methods described below.

KES compression tester (article name, model KES-G5-50) manufactured by Kato Tech Co. was used to measure the compression hardness and the compression recovery.

The measuring conditions were speed: 0.1 cm/sec, compression area: 2 cm², sensitivity: 2 (force meter 200 g/10v), compressive load: 50 gf/cm², and a measuring object is compressed and then LC (compression hardness) and RC (compression recovery) were calculated from a correlation chart between pressure and deformation volume.

The compression hardness is evaluated to be harder as the value of the compression hardness (LC) approaches 1.

The compression recovery (RC) is evaluated to be more recoverable as the value approaches 100%.

### 1.7.4. Entering Property

The pad side absorbent portion 30 to contact the wearer's body in the auxiliary pad 3 is provided with a certain fold starting element in the pad side absorbent portion 30 along the longitudinal direction LD substantially at the center of the pad side absorbent portion 30 in the width direction WD. Specific examples of the folding starting element are described later.

The fold starting element can be disposed, as shown in FIG. 5, so that the basis weight of the ground pulp used as an absorbent body is different locally in the pad side absorbent portion 30, for example. More specifically, the basis weight is 200 g/m² at the region 353 where the fixed portion 4 is formed, and the basis weight is 500 g/m² at the regions 354, 355, and 356 from the fixed portion 4 to the free end portion 32.

Furthermore, as shown in FIG. 5, nondense portions 351 and 352 are formed at the backward region BA of the pad side absorbent portion 30. The nondense portion 351 is formed at the side of the central region CA of the rear region BA of the pad side absorbent portion 30 along the width direction WD, and the nondense portion 352 is formed at the side of the rear edge 230 of the rear region BA of the pad side absorbent portion 30 along the width direction WD.

The nondense portions 351 and 352 are regions where the basis weight of ground pulp is smaller than those of other regions. Additionally, the nondense portions 351, 352 act as a starting point of folding when the absorbent article 1 is folded. Here, the term "near the free end" refers to a region from the outer edge of the handle portion 40 to one-fourth of the total length of the auxiliary pad 3 containing the handle portion 40.

Consequently, when the absorbent article 1 is folded for individual packaging, etc., wrinkles are prevented from occurring due to curvature differences of inner side and outer side of folding.

Moreover, a region 357 having an basis weight of 200 g/m² is formed longitudinally substantially at the center of the pad side absorbent portion 30 in the width direction WD at the region 357 of 80 mm long from a position slightly shifted to the side of the free end portion 32 of the position Z. The region 357 induces a deformation of the auxiliary pad 3 in a worn state.

Preferably, the region 357 is formed at least between the position W where the body excretory part are intended to contact and the nondense portion 352 in the longitudinal direction LD. The vaginal opening as the first excretory area and the anus as the second excretion area are intended to contact between the position Z and the nondense portion 352 in the longitudinal direction LD. More specifically, the region 357 is formed substantially at the center of the pad side absorbent portion 30 in the width direction and from a position slightly shifted from the position Z toward the side of the rear edge 230 to the position of 80 mm in length toward the rear edge 230. In this way, the fold starting element is formed by forming a difference of basis weight in the pad side absorbent portion 30.

The basis weight of ground pulp, in this embodiment, is exemplified by a case in which it is 200 g/m² at the region 357 of 10 mm made so that the center is the middle of the pad side absorbent portion 30 in the width direction WD, and 500 g/m² at the regions 354 and 355 outside therefrom in the width direction WD, for example. By the basis weight being lower at the central region of the pad side absorbent portion 30 in the width direction WD, the central region becomes a fold starting element, and thus the pad side absorbent portion 30 easily enters into the gluteal cleft while projecting to the surface side in the thickness direction as shown in FIG. 27.

It should be noted that an absorbent fiber with a fiber length longer than that of pulp may be disposed at the skin contacting side of ground pulp in order to improve skin contact when the pad side absorbent portion 30 contacts the wearer's body. The construction of the absorbent fiber with a fiber length longer than that of pulp is exemplified by those formed of 60% by mass to 90% by mass of rayon with 3.3 dtex and fiber length 51 mm and 10% by mass to 40% by mass of a cotton, and the basis weight set to 50 g/m² to 500 g/m². By mixing to be layered the absorbent fiber with a fiber length longer than that of pulp formed in this way and disposing at the skin contacting side, skin contact can be improved when the pad side absorbent portion 30 contacts the wearer's body.

Furthermore, another example of the fold starting element formed at the pad side absorbent portion 30 along the longitudinal direction LD is exemplified by a slit 61, as shown in FIG. 28A for example, that is formed to be continuous or intermittent substantially at the center of the pad side absorbent portion 30 in the width direction WD along the longitudinal direction LD of the pad side absorbent portion 30.

In addition, another example is exemplified by a space portion 63, as shown in FIG. 28B for example, that is formed to be continuous or intermittent substantially at the center of the pad side absorbent portion 30 in the width direction WD along the longitudinal direction LD of the pad side absorbent portion 30.

The absorbent core 64 of the pad side absorbent portion 30 in the aforementioned case is formed of an air raid pulp that is made to be a composite of 60:10:30 of a pulp, an absorbent polymer, and a composite fiber having a polypropylene core and a polyethylene sheath. The basis weight of the air raid pulp is exemplified by 200 g/m².

It should be noted that, due to the air raid pulp having a tendency to have a hard feel of skin contact when contacting with skin, a ground pulp or an absorbent fiber with a fiber length longer than that of pulp can be disposed in the air raid pulp at the body contacting side. In a case where a ground pulp or an absorbent fiber with a fiber length longer than that of pulp is disposed, since the pad side absorbent portion 30 is thickened in the thickness direction, it is preferred that a nondense portion 1 mm to 5 mm of a space portion with a certain width is formed rather than the slit 61 described above.

Furthermore, the fold starting element is exemplified by a region having a different fiber basis weight outside of the pad side absorbent portion 30 in the width direction WD that is formed to be continuous or intermittent substantially at the center of the pad side absorbent portion 30 in the width direction WD along the longitudinal direction LD, for example.

For example, first, a case can be exemplified in which the basis weight is higher at the central region of the side absorbent portion 30 in the width direction and the basis weight is lower at the outer region in the width direction WD.

More specifically, as shown in FIG. 28C, a case can be exemplified in which the pad side absorbent portion 30 has a central region 65 formed substantially at the center in the width direction WD and an outer region 66 formed outside of the central region 65 in the width direction WD.

Additionally, in this case, when the difference between the basis weight at the central region 65 and the basis weight at the outer region 66 is within a certain range, the pad side absorbent portion 30 has appropriate bendability and absorbability.

For example, a case can be exemplified in which the difference in basis weight between the central region 65 and the outer region 66 of the pad side absorbent portion in the width direction is 50 g/m² to 1000 g/m², preferably 100 g/m² to 800 g/m², and particularly preferably 150 g/m² to 600 g/m².

Furthermore, the fold starting element is exemplified by a region having a different fiber density outside of the pad side absorbent portion 30 in the width direction WD that is formed to be continuous or intermittent substantially at the center of the pad side absorbent portion 30 to configure the pad side absorbent layer in the width direction WD along the longitudinal direction LD, for example.

For example, first, a case can be exemplified in which the density is higher at the central region 65 of the side absorbent portion 30 in the width direction WD and the density is lower at the outer region 66 in the width direction WD. Next, a case can be exemplified in which the density is lower at the central region of the side absorbent portion 30 in the width direction WD and the density is higher at the outer region in the width direction WD.

More specifically, as shown in FIG. 28C, a case can be exemplified in which the pad side absorbent portion 30 has a central region 65 formed at the middle in the width direction WD and an outer region 66 formed outside of the central region 65 in the width direction WD.

Additionally, in this case, when the difference between the density at the central region 65 and the density at the outer region 66 is within a certain range, the pad side absorbent portion 30 has appropriate bendability and absorbability.

For example, a case can be exemplified in which the difference in density between the central region 65 and the outer region 66 of the pad side absorbent portion 30 in the width direction WD is 0.005 g/cm³ to 0.5 g/cm³, preferably 0.01 g/cm³ to 0.3 g/cm³, and particularly preferably 0.015 g/cm³ to 0.2 g/m³.

More specifically, as shown in FIG. 28C, a case can be exemplified in which the pad side absorbent portion 30 has a central region 65 formed at the middle in the width direction WD and an outer region 66 formed outside of the central region 65 in the width direction WD.

Furthermore, the fold starting element is exemplified by a certain broken line that is formed to be continuous or intermittent by previously doing a fold-processing at substantially the center of the pad side absorbent portion 30 to configure the pad side absorbent layer in the width direction WD along the longitudinal direction LD, for example.

As shown in FIG. 27, the central portion in the width direction WD is made to project to the surface side of the pad side absorbent portion 30 to form a certain broken line by way of a predetermined apparatus and the like. Therefore, the absorbent article 1 can deform so as to fold along the broken line in actual use.

Furthermore, the fold starting element is exemplified by a core portion that is disposed substantially at the middle of the pad side absorbent portion 30 to configure the pad side absorbent layer in the width direction WD along the longitudinal direction LD, for example.

The core portion is exemplified, as shown in FIG. 28D, by a certain compressed portion 67 that is formed to be continuous or intermittent at substantially the middle of the pad side absorbent portion 30 in the width direction WD along the longitudinal direction LD.

When the absorbent body 35 of the pad side absorbent portion 30 includes a ground pulp as a main material, the absorbent body 68 is formed by wrapping a blend body of the ground pulp and an absorbent polymer with a tissue having a basis weight of 15 g/m². It should be noted that the absorbent polymer is exemplified by that having a basis weight at the pad side absorbent portion 30 which is substantially uniformly 15 g/m², for example. The basis weight of the ground pulp is exemplified in this embodiment by 500 g/m², for example.

Additionally, a pin-shape emboss can be formed with a certain interval at the substantially central region of the pad side absorbent portion 30 in the width direction WD. Consequently, a core portion is formed at the substantially central region of the pad side absorbent portion 30 in the width direction WD and the central region becomes a fold starting point, and the pad side absorbent portion 30 is likely to enter into the gluteal cleft while deforming into a convex-shape toward the surface side as shown in FIG. 27.

It should be noted that it is preferred for the embossing process to be performed from the face of the pad side absorbent portion 30 at the side of the base absorbent body 2.

Furthermore, an absorbent fiber with a fiber length longer than that of pulp may be disposed at the skin contacting side of the ground pulp. The configuration of the absorbent fiber with a fiber length longer than that of pulp is exemplified by those formed of 60% by mass to 90% by mass of rayon of 3.3 dtex and a fiber length of 51 mm and 10% by mass to 40% by mass of a cotton, and the basis weight set to 50 g/m² to 500 g/m². By mixing and laminating the absorbent fiber with a fiber length longer than that of pulp formed in this way, and then disposing at the skin contacting side, skin contact can be improved when the pad side absorbent portion 30 contacts the wearer's body.

Furthermore, the core portion of a fold starting element can be similarly exemplified, as shown in FIG. 28E, by a tubular absorbent body 69 that is formed by way of winding a certain non-woven fabric and the like to an outer circumference of a certain fiber, which forms the absorbent body 139 of the pad side absorbent portion 30, thereby forming a tubular shape, for example.

Since the rigidity of the tubular absorbent body 69 is higher (harder) than that of the outer region 70 in the width direction where,the fiber configuring the absorbent core 139 is disposed, the tubular absorbent core 69 becomes a fold starting element to deform as shown in FIG. 27. That is, the substantial middle of the pad side absorbent portion 30 in the width direction WD deforms so as to project toward the skin contacting side (arrow F) opposite to the side of the base absorbent body 2 (not shown). Consequently, it can contact so as to enter into a certain groove of the wearer's body.

Furthermore, another example of the fold starting element can be exemplified by a portion where a hot melt adhesive is linearly coated at a site where the top sheet 331 is doubled at the side of the pad side absorbent portion 30 to contact the base absorbent portion. Since the portion, where the top sheet 331 is doubled and also the hot melt adhesive is coated, has a higher rigidity, the portion becomes a fold starting point.

### 1.8. Use Embodiment

A mode of use of the absorbent article 1 with an auxiliary pad of the present invention is described with reference to FIGS. 11 and 13 to 15.

The first mode of use is one in which the auxiliary pad 3 and the base absorbent body 2 are attached to underwear in the attaching step of the auxiliary pad 3 and the base absorbent body 2.

As shown in FIG. 11, the auxiliary pad 3 is in a state to be put on an upper face of the base absorbent body 2. The auxiliary pad 3 is disposed at the central portion 20 of the base absorbent body 2 along the longitudinal direction LD. The entire of the auxiliary pad 3 and the base absorbent body 2 (absorbent article 1 with an auxiliary pad) is constructed to be deformable into an overall gentle U-shape so as to fit curvatures near the excretory part of the wearer's body.

Then, as shown in FIG. 13, the base absorbent body 2 is disposed at a crotch area of underwear. Additionally, the wings 23A and 23B are folded back so as to wrap around the underwear at the disposed site similarly to conventional absorbent articles. The base absorbent body 2 is fixed to the underwear by the wing side dislocation preventing portions 24A and 24B disposed at a back face of the wings 23A and 23B. Additionally, the pad side engagement portions 37a and 37b of the auxiliary pad 3 are engaged to a certain positions of the base absorbent body 2. Subsequently, the auxiliary pad 3 and the base absorbent body 2 are pulled up to the side of the wearer's body along with underwear in a state in which the auxiliary pad 3 is disposed on the upper face of the base absorbent body 2. Here, due to the auxiliary pad 3 being in a state to be engaged to the central portion 20 of the base absorbent body 2 by the pad side engagement portions 37a and 37b, the auxiliary pad 3 is not spaced apart from the base absorbent body 2 until the engagement is released during the attaching step.

Continuing, as shown in FIG. 15, the user inserts a hand between the wearer's body and the underwear from behind the wearer's body, grips the handle portion 40b, and pulls up the auxiliary pad 3 at the side of the end portion 32b toward the direction of an arrow R so as to pull out to the outer edge of the base absorbent body 2 in the longitudinal direction LD.

Consequently, since engagement by the pad side engaging portion 37b is released, the auxiliary pad 3 is spaced apart from the base absorbent body 2. The auxiliary pad 3, spaced apart from the base absorbent body 2, is moved so as to enter into the gluteal cleft starting from the pad side engaging portion 37a. That is, the auxiliary pad 3 is spaced apart from the base absorbent body 2 at one end side thereof by way of pulling up the handle portion 40b gripped by the user. Additionally, the auxiliary pad 3 is moved so as to contact the excretory part and enter into the gluteal cleft.

Then, as shown in FIG. 15, the auxiliary pad 3 disposed so as to enter the gluteal cleft is engaged at the surface of the base absorbent body 2 by the pad side engaging portion 37b disposed at the side of the end portion 32b. Consequently, the auxiliary pad 3 is maintained at the positional state regulated by the user. More specifically, since the auxiliary pad 3 contacts the wearer's body in a state having a certain tension by the pad side engaging portions 37a and 37b, a certain force always acts on the auxiliary pad 3 in a direction to contact the wearer's body. That is, the auxiliary pad 3 is fixed so as to maintain the condition to contact the wearer's body.

Moreover, according to requirements, the side of the end portion 32a of the auxiliary pad 3 can be pulled up by way of gripping the handle portion 40a so as to pull out the pad side engaging portion 37a toward the direction of an arrow R2 or to the outer edge of the base absorbent body 2 in the longitudinal direction LD. Consequently, the engagement of the pad side engaging portion 37a is released, the auxiliary pad 3 at the side of the end portion 32a is spaced apart from the base absorbent body 2, and the auxiliary pad 3 is disposed to further enter into the gluteal cleft. Additionally, the auxiliary pad 3, disposed so as to enter into the gluteal cleft, is engaged to the base absorbent body 2 by the pad side engaging portion 37a.

When a hook member is used for the pad side engagement portions 37a and 37b, engagement is performed by operating so as to push and contact the pad side engaging portions 37a and 37b to the surface of the base absorbent body 2. When the pad side engaging portions 37a and 37b are pushed to contact the surface of the base absorbent body 2, a plurality of pins, of the hook member configuring the pad side engaging portions 37a and 37b, engages the fiber of non-woven fabric or the like of the base absorbent body 2. Additionally, the auxiliary pad 3 contacts the wearer's body under a condition having a certain tension by the pad side engaging portions 37a, 37b.

The position where the pad side engaging portions 37a and 37b are engaged is more the side of the rear edge 230 than the position of the pad side engagement portions 37a and 37b when the auxiliary pad 3 and the base absorbent body 2 are made into a substantially flat shape. In other words, the position of the engaged portion of the base absorbent body 2 is more to the side of the outer edge (the side of front edge 220 or rear edge 230) in the longitudinal direction LD than the position of the pad side engaging portions 37a and 37b when the auxiliary pad 3 and the base absorbent body 2 are mounted into a substantially flat shape.

Consequently, the auxiliary pad 3 is maintained at the positional state regulated by the user. More specifically, since the auxiliary pad 3 contacts the wearer's body in a state having a certain tension by the pad side engaging portions 37a are 37b, a certain force always acts on the auxiliary pad 3 in a direction to contact the wearer's body. That is, the auxiliary pad 3 is fixed so as to maintain the state of contact with the wearer's body.

By using a hook member with directionality for the pad side engaging portions 37a and 37b, the position can be regulated again even after engaging the auxiliary pad 3 as described above. That is, the engagement by the pad side engaging portions 37a and 37b is released to allow the movement merely by way of the user gripping the handle portion 40a or 40b from behind or the front of the wearer's body, and pulling toward the side of the front edge 220 or rear edge 230. Additionally, the engagement can be performed again similarly as above after the position of the auxiliary pad 3 is regulated.

Furthermore, the back sheet 34, disposed in the auxiliary pad 3 at the side to contact the base absorbent body, prevents leakage of menstrual blood absorbed by the auxiliary pad 3. The engagement of the auxiliary pad 3 is a state in which the pad side absorbent portion 30 at the side of the end portion 32b projects outwardly in the longitudinal direction LD from the rear edge 230 of the base absorbent body 2. Due to the back sheet 34 being disposed at the projecting region, menstrual blood or the like is prevented from adhering to the underwear even when the menstrual blood or the like runs down to the side of end portions 32a and 32b of the auxiliary pad 3.

Here, the auxiliary pad 3, disposed so as to contact the excretory part and to enter into the gluteal cleft, deforms so as to fit the shape of the excretory part or the gluteal cleft.

More specifically, due to the pad side absorbent core 35 of the auxiliary pad 3 having different basis weights depending on the disposed sites, it deforms so as to fit the shape of the excretory part or the gluteal cleft while generating a fold starting point from the portion having a basis weight lower than those of the surroundings. In detail, the distribution is arranged so that the region 357, formed substantially at the middle of the pad side absorbent portion 30 in the width direction WD, has a basis weight of the pad side absorbent core 35 lower than those of the surroundings. Therefore, the region 357 becomes a fold starting point and, as the auxiliary pad 3 is disposed so as to enter into the gluteal cleft, an angle-shaped deformation occurs with an apex of the region 357.

The region 357 is formed so as to correspond with the region from the perineal area to the anus of the wearer's body when the auxiliary pad 3 contacts the gluteal cleft. Accordingly, it is formed so that the auxiliary pad 3 can enter into the deepest portion of gluteal cleft. In this way, since the auxiliary pad 3 deforms correspondingly to the shape of the excretory part and the like as described above, the auxiliary pad 3 is disposed so as to closely contact the excretory part and the like. The auxiliary pad 3 is disposed with no space from the wearer's body. That is, the auxiliary pad 3 is disposed so as to contact the excretory part to directly absorb the menstrual blood and the like, and also so that the flow out of the menstrual blood and the like along the surface of the wearer's body can be suppressed.

Furthermore, the base absorbent body 2 absorbs a certain liquid that cannot be completely absorbed only by the auxiliary pad 3. The certain liquid that cannot be completely absorbed only by the auxiliary pad 3 is absorbed by the base side absorbent core 28 of the base absorbent body 2 through the portion where the auxiliary pad 3 and the base absorbent body 2 contact. The certain liquid, absorbed by the base absorbent body 2, is inhibited to soak through to the underwear by the liquid impermeable back sheet 29 and held by the base side absorbent core 28 of the base absorbent body 2.

The second mode of use can exemplified by a mode of use in which the pad side engagement portion 37b is engaged to the side of underwear after regulating the position of the auxiliary pad 3 when attaching the auxiliary pad 3.

The following steps are similar to those of the first embodiment, i.e. the auxiliary pad 3 and the base absorbent body 2 are disposed at a certain position of underwear, the auxiliary pad 3 of the base absorbent body 2 is disposed at a certain position, and the position of the auxiliary pad 3 is regulated by pulling the handle portion 40b toward the arrow R.

After regulating the position of the auxiliary pad 3, when fixing at the regulated position, the pad side engagement portion 37b can be engaged to the side of inner side or outer side of underwear by folding back the auxiliary pad 3. Consequently, the auxiliary pad 3 enters a state of being hanging to a side of underwear.

In accordance with this embodiment, the user can engage the auxiliary pad 3 at a chosen position of the base absorbent body 2 or underwear, etc. to adjust the position so as to fit the wearer's body while matching the body shape, etc. of the user by the absorbent article 1 with an auxiliary pad including the base absorbent body 2 and the auxiliary pad 3 able to be spaced apart from the base absorbent body. Therefore, liquid such as an excretory substance can be prevented from leaking. Furthermore, since the auxiliary pad 3 can match the shapes of gluteal cleft or the positions of the excretory part that are respectively different according to users, gaps from the wearer's body can be eliminated and liquid such as an excretory substance can be prevented from leaking.

Furthermore, the absorbent article 1 with an auxiliary pad can increase the amount of absorbent core as an absorbent article by having the base absorbent body 2 and can make the base absorbent body 2 absorb even when an amount of liquid that cannot be completely absorbed by the auxiliary pad 3 is evacuated; therefore, leak thereof can be prevented. Additionally, the user can be provided with a secure feeling with no fear of leaking.

In accordance with this embodiment, the absorbent article 1 with an auxiliary pad includes the base absorbent body 2 and the auxiliary pad 3 separable from the base absorbent body 2. Since the auxiliary pad 3 is separable from the base absorbent body 2, there arises no problem by way of releasing the engagement from the base absorbent body 2 and exchanging only the auxiliary pad 3 in a case that only the auxiliary pad 3 is soiled. Consequently, since the amount of waste can be decreased, an effect on the natural environment can be lowered.

In accordance with this embodiment, the auxiliary pad 3 includes a liquid permeable sheet also at the side of the auxiliary pad 3 to contact the base absorbent body 2. Consequently, the liquid once absorbed by the auxiliary pad 3 can transfer from the portion, where the auxiliary pad 3 contacts the base absorbent body 2, to the side of the base absorbent body 2.

In accordance with this embodiment, the auxiliary pad 3 includes the pad side engagement portions 37a and 37b at both ends thereof. Consequently, it can engage the base absorbent body 2, an inner or outer face of the underwear, or the wearer's body to follow motions of the wearer's body even after adjusting the position so as to fit the wearer's body when attaching. Additionally, a leak can be prevented that is derived from dislocation of the auxiliary pad 3 during attaching.

In accordance with this embodiment, the auxiliary pad 3 can deform by making basis weight to be different for various portions of the pad side absorbent body 35 depending on their positions and using the portions with a difference in basis weights as the starting point. More specifically, the auxiliary pad 3 can deform to fit the shapes of gluteal cleft or the shapes of the excretory part. Furthermore, deformation occurring from other potions than the portion with the difference in basis weight as the starting point can be suppressed. Consequently, the auxiliary pad 3 can stably contact closely with the wearer's body with no space and suppress leakage.

### 2. Other Embodiments

The second to the seventh embodiments are explained with reference to FIGS. 16 to 22. The second and third embodiments are other embodiments in terms of the pad side absorbent portion 30, and the fourth and fifth embodiments are other embodiments in terms of the handle portions 40a and 40b. Furthermore, the sixth and seventh embodiments are other embodiments in terms engagement means. In addition, in the following embodiments, portions with no particular explanation are similar to those of the first embodiment and are assigned the same number as long as similar with the first embodiment.

### 2.1. Second Embodiment

As shown in FIG. 16, the auxiliary pad 3A in the second embodiment is different from the first embodiment in terms of the aspect of the pad side absorbent portion 30A of the auxiliary pad 3A. FIG. 16A is a sectional view of the auxiliary pad 3A in the longitudinal direction LD; FIG. 16B is a cross-sectional view at (i) of FIG. 16A; FIG. 16C is a cross-sectional view at (ii) of FIG. 16A; and FIG. 16D is a cross-sectional view at (iii) of FIG. 16A.

The mode of the pad side absorbent portion 30A of the auxiliary pad 3A in the second embodiment is that the surface layer 33 is formed of only the top sheet 331 and disposed so as to wrap the pad side absorbent core 35 up to both edges of the back sheet 34A in the longitudinal direction LD. That is, the back sheet 34A is disposed on the outermost face at the side of the pad side absorbent core 35 to contact the base absorbent body 2 rather than the top sheet 331A. In this case, the material to form the back sheet 34A may be a similar material to that of the top sheet 331A.

Furthermore, the pad side engagement portions 37a and 37b are respectively disposed at the end portions of the handle portions 40aA and 40bA along the width direction WD.

The handle portions 40aA and 40bA are formed so that the handle portion surface sheets 42aA and 42bA and the pad side engagement portions 37a and 37b are disposed to be layered at the regions 38a and 38b formed of only the top sheet 331A and the back sheet 34A, and by pressure-bonding process. Furthermore, the handle portion surface sheets 42aA and 42bA are preferably formed with the same size of the regions 38a and 38b formed of only the top sheet 331A and the back sheet 34A, or may be formed so that the handle portion surface sheets 42aA and 42bA extend outwardly.

The material to form the handle portion surface sheets 42aA and 42bA is preferably that having certain rigidity, and is exemplified by an SMS non-woven fabric.

In the first embodiment, although the regions having various basis weights depending on the positions are formed in the pad side absorbent core 35, the pad side absorbent core 35 may be formed with a constant basis weight without being limited thereto.

The handle portions 40aA and 40bA can be imparted with rigidity higher than that of the handle portions 40aA and 40bA in the first embodiment by including the handle portion surface sheets 42aA and 42bA. Furthermore, the engaging position can be at the underwear in addition to the base absorbent body 2, since both of the pad side engagement portions 37a and 37b are disposed at the side of the handle portions 40aA and 40bA.

### 2.2. Third Embodiment

As shown in FIGS. 17A, 17B, 17C and 17D, the auxiliary pad 3B in the third embodiment is different from the first embodiment in terms of the mode of the pad side absorbent portion 30B of the auxiliary pad 3B. FIG. 17A is a sectional view of the auxiliary pad 3B in the longitudinal direction LD; FIG. 17B is a cross-sectional view at (iv) of FIG. 17A; FIG. 17C is a cross-sectional view at (v) of FIG. 17A; and FIG. 17D is a cross-sectional view at (vi) of FIG. 17A.

The mode of the pad side absorbent portion 30B of the auxiliary pad 3B in the third embodiment is that, as shown in FIG. 17C, only the top sheet 331B is disposed at the skin contacting side of the pad side absorbent body 35B as the surface layer 33 and the back sheet 34B is disposed on the entire face at the side to contact the base absorbent body 2. Additionally, the pad side absorbent portion 30B is formed so that the portions, at the both edges of the pad side absorbent body 35B in the width direction and where formed only of the top sheet 331B and the back sheet 34B, are treated by a pressure-bonding process along the longitudinal direction LD. In this case, the material to form the back sheet 34B may be a similar material to that of the top sheet 331B.

Furthermore, the handle portions 40aB and 40bB are formed so that the handle portion surface sheets 42aB and 42bB and the handle portion back sheets 43aB and 43bB are disposed on the regions 38a and 38b that are formed only of the top sheet 331B at both ends of the pad side absorbent portion 30B in the longitudinal direction LD and the back sheet 34B. More specifically, the regions 38a and 38b are disposed to be layered so as to be pinched by the handle portion surface sheets 42aB and 42bB and the handle portion back sheets 43aB and 43bB.

Moreover, the pad side engaging portions 37a and 37b are disposed at the side of the handle portion back sheets 43aB and 43bB to contact the base absorbent body 2. Additionally, the regions 38a and 38b formed only of the top sheet 331B and the back sheet 34B disposed to be layered, the handle portion surface sheets 42aB and 42bB, the handle portion back sheets 43aB and 43bB, and the pad side engagement portions 37aB and 37bB are bonded, for example, by an HMA (hot melt adhesive) into a substantially plate-shape.

The material for forming the handle portion surface sheets 42aB and 42bB may be the material forming the handle portion surface sheets 42aA and 42bA in the second embodiment. Furthermore, the handle portion back sheets 43aB and 43bB are also similar.

### 2.3. Fourth Embodiment

As shown in FIG. 18, the auxiliary pad 3C in the fourth embodiment is different from the first embodiment in terms of the mode of the handle portion 40C and the pad side engagement portion 37 at both ends of the pad side absorbent portion 30C of the auxiliary pad 3C in the longitudinal direction LD.

In the auxiliary pad 3C of the fourth embodiment, one end of an elastic member 36C is disposed at the region 38C formed only of the top sheet 331 and the back sheet 34 at both ends in the longitudinal direction LD. The handle portion 40bC is disposed at the other end of the elastic member 36C. The pad side engagement portion 37bC is disposed at the handle portion 40bC.

The pad side engagement portion 37bC is disposed at the side of the handle portion 40bC to contact the base absorbent body 2. Additionally, the pad side engagement portion 37bC, the elastic member 36C, and the handle portion surface sheet 42bC are disposed to be layered sequentially from the side to contact the base absorbent body 2 and treated by a pressure-bonding process. It should be noted that the pad side engagement portion 37bC may be disposed at the skin contacting side of the handle portion 40bC. By disposing the pad side engagement portion 37bC at the skin contacting side of the handle portion 40bC, the handle portion 40bC can be folded back and engaged to the base absorbent body 2 or an inner face of the underwear. Furthermore, in this case, the pad side engaging portion 37bC may be formed from a sticky material. By configuring from a tacky material, it is possible to fix by contacting the skin of users.

The material for forming the elastic member 36C can be exemplified by a stretchable non-woven fabric, those prepared by laminating a non-woven fabric on a stretchable material such as thread rubber so as to sandwich thereof, and the like. Furthermore, the stretchable non-woven fabric may be non-woven fabrics of which the fiber itself is a stretchable fiber or other various materials.

In addition, a stretchable non-woven fabric prepared by gear stretching can be exemplified as a preferable elastic material. More specifically, it can be formed by a spunbond non-woven fabric that is a mixture of a polyurethane long fiber and a polypropylene long fiber. For example, it can be obtained by mixing a polyurethane fiber and a polypropylene fiber are mixed in a ratio of 50/50 to form a non-woven fabric having an basis weight of 35 g/m², and performing gear-stretching process on the resulting non-woven fabric in a stretching range of two times in the longitudinal direction LD.

The elastic member 36C and the handle portion 40bC may be disposed at both end portions of both ends of the pad side absorbent portion 30C or may be disposed at either one. It should be noted that, in this embodiment, although only the side of the end portion 32b is explained, the side of the end portion 32a has a similar configuration in a case where both ends of the pad side absorbent portion 30C are used for disposing.

### 2.4. Fifth Embodiment

As shown in FIG. 19, the auxiliary pad 3C in the fifth embodiment is different from the first embodiment in terms of the mode of the pad side engaging portion 37D of the auxiliary pad 3D. An elastic member 36D for a buffer region is disposed at the pad side engaging portion 37bD of the auxiliary pad 3D in the fifth embodiment.

The elastic member 36D is disposed at the side of the handle portion 40b to contact the base absorbent body so as to straddle across the handle portion 40b and the pad side absorbent portion 30. The elastic member 36D is disposed at the side of the pad side absorbent portion 30 to contact the base absorbent body 2 so as to straddle across from near the end portion of the pad side absorbent portion 30 to near the end portion of the handle portion 40b at the side of the pad side absorbent portion 30. The elastic member 36D is fixed so as to traverse in the width direction WD both ends of the auxiliary pad 3 in the longitudinal direction LD. Both ends of the auxiliary pad 3D in the longitudinal direction LD to fix the elastic member 36D are fixed by an HMA (hot melt adhesive) or by performing an embossing process.

The pad side engaging portion 37D is disposed at an inner side in the longitudinal direction LD from the edge of the pad side absorbent core 35 at the side to contact the base absorbent body 2 of the elastic member 36D.

It should be noted that the elastic member 36D may be disposed only at the side of the pad side absorbent portion 30 to contact the base absorbent body 2 or only at the handle portion 40b rather than to be disposed so as to straddle across the pad side absorbent portion 30 and the handle portion 40b. Additionally, the material to be used for the elastic member 36D may be similar as the elastic member in the fourth embodiment.

The elastic member 36D may be disposed at end portions of both ends of the pad side absorbent portion 30 or may be disposed at either one. It should be noted that, in this embodiment, although only the side of the end portion 32b is explained, the side of the end portion 32a has a similar configuration when both ends of the pad side absorbent portion 30 are used for the deposition.

### 2.5. Sixth Embodiment

As shown in FIGS. 20A and 20B, the auxiliary pad 3E and the base absorbent body 2E according to the sixth embodiment are different from the first embodiment in terms of the mode of the engaging means. FIG. 20A is a back side view of the auxiliary pad 3E and FIG. 20B is a plan view of the base absorbent body 2E.

In the sixth embodiment, pad side engaged portions 325a and 325b are disposed at the auxiliary pad 3E and base side engaged portions 237 and 237b are disposed at the base absorbent body 2E.

As shown in FIG. 20A, the pad side engaged portions 325a and 325b are disposed near the end portions 32a and 32b of both ends of the auxiliary pad 3E in the longitudinal direction LD at the side of the auxiliary pad 3E to contact the base absorbent body 2E. The pad side engaged portions 325a and 325b are formed form a loop material, film, etc., for example, and become a region where the base side engaging portions 237a and 237b are engaged. Furthermore, the material forming the pad side engaged portions 325a and 325b can be selected from materials suited so as to combine the material used for the base side engaging portions 237a and 237b.

The base side engaging portion 237b of the base absorbent body 2E contacts the pad side engaged portion 325b at the side of the handle portion 40b. The side to dispose the base side engaging portion 237b is intended to be disposed facing the buttocks side of the wearer's body. In detail, the pad side engaged portion 325b is a region engaged after the handle portion 40b of the auxiliary pad 3E is gripped, and the position of the auxiliary pad 3E is adjusted. Accordingly, it is preferred that the area of the pad side engaged portions 325b is larger than that of the pad side engaged portion 325a.

It should be noted that, in a case where the base side engagement portions 237a and 237b are formed of a hook member, the entire face can be used as a base side engaged portion by way of forming the face of the pad side engaging portion 30E at the side of the base absorbent body 2E by a non-woven fabric.

Preferably, the pad side engaged portions 325a and 325b are disposed along a distance of less than 100 mm, and more preferably less than 70 mm in the longitudinal direction LD from the edges 32a and 32b of the pad side absorbent portion 30 to the end portions of the pad side engaged portions 325a and 325b at the side of the handle portions 40a and 40b. If the separation distance is 100 mm or more, it may be difficult to separate the engagement when adjusting the position of the auxiliary pad 3E.

As shown in FIG. 20B, in the base absorbent body 2E, the base side engaging portions 237a and 237b are respectively disposed at certain positions at the side of the front edge 220 and at the side of the rear edge 230 of the central portion 20 in the longitudinal direction LD of the base absorbent body 2E. Furthermore, an elastic member 82E for a buffer region is disposed in the base absorbent body 2 at the side of the rear edge 230. The base side engaging portion 237b is disposed substantially at the middle in the longitudinal direction LD and in the width direction WD of the elastic member 82E at the side to contact the auxiliary pad 3E. Furthermore, the material to be used for the elastic member 36C may be similar to the elastic member 36C according to the fourth embodiment.

As shown in FIG. 21, the auxiliary pad 3E is packed by a substantially rectangular packaging sheet 50E. Additionally, engaging tape 51 is disposed substantially at the middle in the width direction WD of one end portion in the longitudinal direction LD at one face side of the packaging sheet 50E.

The packaging sheet 50E can be formed by an SMS non-woven fabric, for example. Additionally, the auxiliary pad 3E is formed substantially at the middle of the packaging sheet 50E in the width direction WD along the longitudinal direction LD. In the packaging sheet 50E, engaging portions 537a and 537b are disposed on the packaging sheet 50E of another side at the position to contact the pad side engaged portions 325a and 325b of the auxiliary pad 3E. Additionally, the auxiliary pad 3E is fixed to the packaging sheet 50E by engaging the pad side engaged portions 325a and 325b to the engaging portions 537a and 537b.

After engaging the auxiliary pad 3E to the packaging sheet 50E, the packaging sheet 50E along with the auxiliary pad 3E is folded to the side of the auxiliary pad 3E in the order of the first folding P and the second folding Q as shown in FIG. 22, and then engagement is carried out by the engaging tape 51 in the folded state. Moreover, the side periphery in the folded state is sealed by a pressure-bonding process.

### 2.6. Seventh Embodiment

As shown in FIGS. 22A and 22B, the auxiliary pad 3F and the base absorbent body 2F according to the seventh embodiment are different from the first embodiment in terms of the mode of the engagement means. FIG. 22A is a back side view of the auxiliary pad 3F, and FIG. 22B is a plan view of the base absorbent body 2F.

A pad side engaging portion 37aF and a pad side engaged portions 325F are disposed at the auxiliary pad 3F. Furthermore, a base side engaged portion 25aF and a base side engaging portion 237F are disposed at the base absorbent body 2F.

It is preferred for the pad side engaging portion 237F to be disposed substantially at the middle of the elastic member 82F at the side to contact the auxiliary pad 3F. Furthermore, the material to be used for the elastic member 82F may be similar to that of the elastic member 36C according to the fourth embodiment.

The pad side engaged portions 325F and the base side engaged portion 25aF can be formed by a loop material, for example. More specifically, for example, the loop material can be exemplified by a liquid impermeable film on which a long fiber is adhered in a loop-shape. By using the loop material in the side of the pad side engaged portion 325F not only as the engaging means, liquid leakage from the auxiliary pad 3F can be decreased.

The pad side engaged portions 37a and 37b that are disposed at the auxiliary pad 3 as an engaging means to engage the base absorbent body 2 and the auxiliary pad, and the surface sheet 27 of the base absorbent body 2 as an engaged portion are explained in the embodiments described above, but is not limited thereto. For example, as shown in the sixth embodiment, an engaged portion may be disposed at the side of the auxiliary pad 3 and an engaging portion may be disposed at the side of the base absorbent body 2. Furthermore, as shown in the seventh embodiment, one engaging portion may be disposed at the auxiliary pad 3 and another engaging portion may be disposed at the base absorbent body 2. In addition, as in the sixth and seventh embodiments, a sheet-shape member may be disposed at the surface of the base absorbent body 2. Moreover, the sheet-shape member may be stretchable. When the engaged portion is a stretchable sheet-shape member, the engaged portion is stretched when the wearer's body or underwear moves, thus becoming a buffer region to prevent the engaging portion from separating from the engaged portion. Furthermore, as the fifth embodiment, an elastic member may be disposed at the side of the engagement portion. More specifically, the elastic member may be disposed between the base absorbent body 2 or the auxiliary pad 3 and the engaging portion.

In the embodiments described above, the pad side engaging portions 37a and 37b are engaged to the base absorbent body 2 in the worn state, but is not limited thereto, and it may be possible to engage to not only inside but also outside of underwear as a garment disposed outside the absorbent article. More specifically, the pad side engaging portions 37a and 37b are enabled to engage not only to inside, but also the outside of underwear in the state where the absorbent article 1 is disposed between a wearer's body as the attaching object and the underwear as a garment disposed so as to cover the wearer's body. In this case, the pad side engaging portions 37a and 37b are disposed at a farther outerward side of the base absorbent body 2 in the longitudinal direction LD than the position to be disposed in the case of being engaged to the base absorbent body 2 described above.

In the embodiments described above, the base absorbent body 2 has gathers 21A and 21B or a compressed groove 22, but which is not limited thereof, and may not have gathers 21A and 21B or the compressed groove 22. Furthermore, the base absorbent body 2 has six compressed grooves 22 at the central portion with substantially equal spacing in the width direction, but is not limited thereto. For example, the base absorbent body 2 may have a circular compressed groove that extends in the longitudinal direction LD, in which the compressed groove is formed so that the portion corresponding to the position Z in the width direction WD sinks to inside in the width direction WD as well as a shallow curved compressed groove that is formed outside the portions where the former grooves are formed to sink to inside in the width direction WD.

Furthermore, in the embodiments, the base side absorbent body 28 in the base absorbent body 2 has nondense portions 351 and 352 and basis weight-variable regions 353, 354, 355, 356 and 357, as shown in FIG. 26, but the positions to form them and the basis weights are not limited thereto, and other positions or different basis weights are allowable. In addition, the base side absorbent core 28 may have a uniform basis weight at the entire region.

Moreover, the base absorbent body 2 may be a commercially available conventional sanitary napkin having other configurations than those of the base absorbent body 2 described above.

Furthermore, the base absorbent body 2 may be a commercially available conventional sanitary napkin having other configurations than those of the base absorbent body 2 described above. The entire shape of the base absorbent body 2 is not limited to a substantially rectangular shape, and any shape with a longitudinally longer length is allowable such as ellipse.

### 3. Examples

### 3.1. Example 1

An auxiliary pad 3 was prepared so that a ground pulp with a certain basis weight was made 35 mm long in the width direction WD, which was wound by an air through non-woven fabric of 35 g/m², and the overlapped portions were adhered by a hot melt adhesive. The auxiliary pad 3 was measured with respect to the flexural rigidity and compression hardness in accordance with the methods described, in a manner that the auxiliary pad 3 is bent at a certain position in the longitudinal direction LD so as to form a broken line parallel to the width direction WD. Furthermore, the samples of "pressed" in Table 3 shown below indicate those in which the resulting auxiliary pad 3 was treated by a pressure-bonding process using a press with a flat surface.

In addition, a sensory assessment test was performed on the auxiliary pad 3. In the sensory assessment method, ten women having a hip size of 85 to 95 cm were requested to respectively wear the samples shown below as a test to evaluate comfortability with the wearer's body and a secure feeling during attachment by scoring with a maximum of ten points. The use order was random. Additionally, the results were classified based on the evaluation average of ten women, such as a double circle: 8 or higher, a circle: below 8 and 6 or higher, a triangle: below 6 and 4 or higher, and a cross mark: below 4.

**[Table 1]**

| Sample | | P1 | P2 | P3 | P4 | P5 | P6 | P7 | G |
|---|---|---|---|---|---|---|---|---|---|
| Basis Weight of Absorbent Core (Ground Pulp) | | 200 | 300 | 450 | 500 | 600 | 800 | 1000 | 800 |
| Press of Absorbent Core | | - | - | - | - | - | - | - | pressed |
| Thickness | | 5.6 | 7.8 | 11.2 | 12.3 | 14 | 17.8 | 22.4 | 11.9 |
| Value of Flexural Rigidity | | 0.09 | 0.16 | 0.4 | 0.48 | 0.66 | 1.2 | 1.72 | - |
| Compression Hardness (LC) | | 0.58 | 0.61 | 0.68 | - | 0.74 | 0.73 | - | 0.83 |
| Sensory Assessment | comfortability | ⊚ | ⊚ | ⊚ | ⊚ | ⊚ | ○ | △ | △ |
| | secure feeling | △ | ○ | ⊚ | ⊚ | ⊚ | ○ | × | - |

When the auxiliary pad 3 was prepared with a lower basis weight, the comfortability was higher, but score for feeling was lacking, and liquid-absorbing capacity was less, resulting in a lower score for secure feeling.

When the auxiliary pad 3 was prepared with a higher basis weight, the result was inferior for comfortability and secure feeling. This is because a rigid feeling was generated and users felt a gap caused by the fact that a larger material amount makes close contact with the gluteal cleft impossible.

Furthermore, the result of the comfortability in the sensory assessment was such that samples P1 to P6 were favorable. In addition, the result of the secure feeling was such that samples P2 to P6 were favorable. Sample P7, of which the value of flexural rigidity was higher than 1.7 N, had an inferior evaluation result in terms of comfortability as well as secure feeling. Furthermore, sample G had a compression hardness of above 0.8 (-), but sample G had a result of inferior familiarity.

### 3.2. Example 2

A base absorbent body 2 was prepared in a way so that a ground pulp was wound by a tissue of 15 g/m², to which an air through non-woven fabric of 35 g/m² was adhered by a hot melt adhesive, followed by forming a compressed groove 22 to consolidate them. Then, a base absorbent body 2 was prepared by laminating an impermeable film of 23 g/m² to the back side. The length of the base absorbent body 2 was 75 mm in the width direction WD.

Furthermore, a pattern of two different compressed grooves 22 was formed. The compressed groove pattern A is similar to the pattern of the compressed groove 22 in the first embodiment. Additionally, the measurement site is a region having a center at the position Z in the longitudinal direction LD where six compressed grooves 22 are formed with substantially equal spacing in the width direction. Furthermore, the compressed groove pattern B is formed in the base absorbent body 2 to have a circular compressed groove that extends in the longitudinal direction LD, in which the compressed groove is formed so that the portion corresponding to the position Z in the width direction WD sinks to inside in the width direction WD as well as a shallow curved compressed groove that is formed outside the portions where the former groove is formed to sink to inside in the width direction WD. That is, four compressed grooves are formed at the measurement site in the width direction WD. Additionally, the absorbent articles with the respective compressed groove patterns were measured for their flexural rigidity in a case where the bending was performed so as to form a broken line parallel with the width direction WD. The measurement method of the flexural rigidity was as described above.

**[Table 2]**

| Sample | H1 | H2 | H3 | H4 |
|---|---|---|---|---|
| Basis Weight of Absorbent Core (Ground Pulp) | 300 | 400 | 500 | 750 |
| Pattern of Compressed Groove | A | A | B | B |
| Thickness at Central Portion | 3.26 | 3.89 | 6.14 | 10.4 |
| Flexural Rigidity | 0.24 | 0.75 | 0.32 | 1.3 |

Absorbent articles 1 were prepared by a combination of samples P6 and H1 and a combination of samples P7 and H2 prepared in Example 1, and an attachment test was carried out.

The result was that the auxiliary pad 3 resulted in dislocation since the base absorbent body 2 has a higher rigidity and is weaker, and thus the auxiliary pad 3 cannot follow the curvature of the main body in the step of attaching the absorbent article 1 to the underwear.

### 3.3. Example 3

Handle portions 40 were prepared in the configuration shown in the table below, and each thereof was subjected to the sensory assessment with respect to hardness (flexural rigidity), flexure recovery, and feeling of holding.

A sensory test was performed and evaluated with respect to hardness (flexural rigidity), flexure recovery, and feeling of holding by ten women. The test was performed for the samples in random order. The evaluation was an average of scores of each item that was scored with a maximum of ten points. The results were classified based on the average score, such as a double circle: 8 or higher, a circle: below 8 and 6 or higher, a triangle: below 6 and 4 or higher, and a cross mark: below 4.

### Evaluation Method

In the hardness evaluation method, the preferable hardness was made into a score when gripping the handle portion 40 of the sample.

With respect to the flexure recovery, the handle portion 40 of a sample was doubled into a folded state, and allowed to stand at 20°C and 60% humidity for 1 hour in a state loaded at 20 g/m². Subsequently, the load was released and the sample was further allowed to stand at the same humidity conditions described above for 1 hour, and then a score was given for the hardness at the end portion of the fold line in terms of whether an uncomfortable feeling generates therefrom.

The feeling of holding at the handle portion 40 was evaluated with respect to the thickness of the handle portion 40. More specifically, a score was given for whether the handheld feeling corresponds to a desirable thickness when gripping the handle portion 40. "HMA" as described in Table 3 below is an abbreviation for hot melt adhesive.

**[Table 3]**

| | Hardness | | Recovery | | Thickness: Feeling of holding | | |
|---|---|---|---|---|---|---|---|
| Test sample | B | Sensory | 2HB | Sensory | Thickness | Sensory | Configuration |
| 1 | 0.049 | × | 0.0961 | ⊚ | 0.912 | ○ | A through-air non-woven fabric having a basis weight of 35 g/m² |
| 2 | 0.1943 | ○ | 0.439 | ⊚ | 1.78 | ⊚ | Two through-air non-woven fabrics having a basis weight of 35 g/m² laminated with HMA |
| 3 | 0.3321 | ⊚ | 0.765 | ⊚ | 3.56 | ○ | Four through-air non-woven fabrics having a basis weight of 35 g/m² laminated with HMA |
| 4 | 0.3565 | ⊚ | 1.0248 | ⊚ | 1.75 | ⊚ | A through-air non-woven fabric having a basis weight of 35 g/m² and a film having a basis weight of 23 g/m² laminated with HMA and folded into three |
| 5 | 0.4507 | ⊚ | 0.5643 | ⊚ | 0.487 | △ | Two SMS non-woven fabrics having a basis weight of 35 g/m² laminated with HMA |
| 6 | 0.5685 | ⊚ | 1.0863 | ⊚ | 0.741 | ○ | Three SMS non-woven fabrics having a basis weight of 35 g/m² laminated with HMA |
| 7 | 0.7795 | ⊚ | 2.914 | ⊚ | 0.906 | ⊚ | Four SMS non-woven fabrics having a basis weight of 35 g/m² laminated with HMA |
| 8 | 1.0865 | ○ | 6.3762 | ○ | 1.367 | ⊚ | Six SMS non-woven fabrics having a basis weight of 35 g/m² laminated with HMA |
| 9 | 1.2719 | △ | 10.7168 | × | 1.567 | ⊚ | Seven non-woven fabrics having a basis weight of 35 g/m² laminated with HMA |

A result was obtained that the hardness of the handle portion 40 is preferably within a range of 0.1 to 1.2 (10⁻⁴ N·m²/m). Furthermore, a result was obtained that the flexure recovery of the handle portion 40 is preferably no higher than 10 (10⁻² N·m/m). Moreover, a result was obtained that the thickness of the handle portion 40 is preferably within a range of 0.5 to 4 mm.

## Claims

1. An absorbent article, comprising:
a base absorbent article of substantially oblong shape; and
an auxiliary pad at a skin contacting side of the base absorbent article, at least one end portion of the auxiliary pad being engaged to be separable from the base absorbent article,
wherein the auxiliary pad comprises:
a pad side absorbent layer,
a liquid permeable sheet disposed at a surface of the pad side absorbent layer,
a pad side engaging portion, disposed at a skin noncontacting side of the auxiliary pad which is opposite to the skin contacting side, that engages the base absorbent article and the auxiliary pad, and
a substantially plate-shaped handle portion disposed on at least one end portion in the longitudinal direction of the auxiliary pad.

2. The absorbent article according to claim 1, wherein flexural rigidity of the auxiliary pad toward the skin contacting side is 0.05 to 1.7 N.

3. The absorbent article according to claim 1, wherein compression hardness (LC) of the auxiliary pad is 0 to 0.8 (-) at the surface of the skin contacting side.

4. The absorbent article according to claim 1, wherein the auxiliary pad has a first region, extending in the longitudinal direction, at substantially a middle in a width direction, which is perpendicular to the longitudinal direction of the auxiliary pad, and
the first region is different in rigidity from a second region, formed at both sides of the first region in the width direction so as to extend in the longitudinal direction.

5. The absorbent article according to any one of claims 1 to 4, wherein the pad side engaging portion comprises a first engaging portion and a second engaging portion, and
at least one of the first engaging portion and the second engaging portion is configured of a mechanical engaging material.

6. The absorbent article according to claim 5, wherein at least one of the first engagement portion and the second engagement portion is a hook member,
the hook member
comprising a plurality of pins and a base portion to support the plurality of pins,
each of the pins being formed so that at least a portion of each pin tilts from the base portion, and
the hook member having a higher engaging force when each of the pins is slid toward a direction tilted from the base portion than an engaging force when each of the pins is slid toward the opposite direction.

7. The absorbent article according to claim 6, wherein a tip end of each of the pins is disposed so as to face the inside of the auxiliary pad in the longitudinal direction.

8. The absorbent article according to any one of claims 5 to 7, wherein
at least one of the first engaging portion and the second engaging portion is restricted in movement toward inside in the longitudinal direction and not restricted in movement toward outside in the longitudinal direction, in a state where the first engaging portion and the second engaging portion are respectively engaged at one face of the base absorbent article.

9. The absorbent article according to any one of claims 5 to 8, wherein at least one of the first engaging portion and the second engaging portion
has a shear strength of at least 6 N against an inward force in the longitudinal direction, and
a shear strength of no higher than 6 N against an outward force in the longitudinal direction.
